(19) Europäisches Patentamt
European Patent Office
Office européen des brevets

(11) EP 2 103 653 A1

(12) **EUROPEAN PATENT APPLICATION**
published in accordance with Art. 153(4) EPC

(43) Date of publication:
**23.09.2009 Bulletin 2009/39**

(21) Application number: **07860290.1**

(22) Date of filing: **27.12.2007**

(51) Int Cl.:
***C08L 65/00*** *(2006.01)* ***C08G 61/12*** *(2006.01)*
***C08K 5/3432*** *(2006.01)* ***C09K 11/06*** *(2006.01)*
***H01L 51/50*** *(2006.01)*

(86) International application number:
**PCT/JP2007/075068**

(87) International publication number:
**WO 2008/081852 (10.07.2008 Gazette 2008/28)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **27.12.2006 JP 2006351847**

(71) Applicants:
- **Sumitomo Chemical Company, Limited**
  **Tokyo 104-8260 (JP)**
- **Sumation Co., Ltd.**
  **Chuo-ku**
  **Tokyo 104-8260 (JP)**

(72) Inventors:
- **AKINO, Nobuhiko**
  **Ibaraki (JP)**
- **DELBECQ, Frederic**
  **Tsukuba-shi**
  **Ibaraki (JP)**

(74) Representative: **Duckworth, Timothy John**
**J.A. Kemp & Co.,**
**14 South Square,**
**Gray's Inn**
**London WC1R 5JJ (GB)**

# (54) COMPOSITION AND LIGHT-EMITTING ELEMENT COMPRISING THE COMPOSITION

(57) Disclosed is a composition comprising a compound having a pyrazine ring structure and a phosphorescent compound, wherein the compound having a pyrazine ring structure has a pyrazine ring structure represented by the general formula (1), (2) or (3):

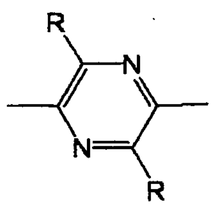
(1)

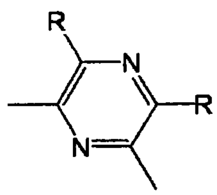
(2)

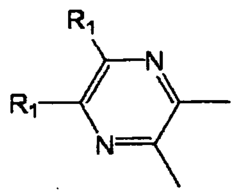
(3)

wherein R and $R_1$ independently represent a hydrogen atom or a univalent group and multiple R's and multiple $R_1$'s may be the same as or different from one another. Also disclosed is a polymer having a residue derived from the phosphorescent compound and the pyrazine ring structure.

EP 2 103 653 A1

## Description

### FIELD OF THE INVENTION

The present invention relates to a composition comprising: a compound having a pyrazine ring structure; and a phosphorescence-emitting compound, and to a light-emitting device comprising the composition.

### BACKGROUND ART

A light-emitting device comprising a compound that exhibits light emission from a triplet excited state (hereinafter, also referred to as a "phosphorescence-emitting compound") used as a light-emitting material for a light-emitting layer therein has been known to have high luminous efficiency. When the phosphorescence-emitting compound is used in a light-emitting layer, a composition obtained by adding the compound to a matrix is usually used as a light-emitting material. A polymer such as polyvinylcarbazole can be used preferably as the matrix, because a thin film can be formed by application (PATENT DOCUMENT 1). However, such a polymer has a problem that an electron injection is difficult because the lowest unoccupied molecular orbital (hereinafter, also referred to as "LUMO") is high.
On the other hand, when a conjugated polymer such as polyfluorene is used as the matrix, electrons can be injected relatively easily because of its low LUMO. However, such a conjugated polymer has a small lowest triplet excitation energy and is therefore unsuitable particularly for use as a matrix for light emission with a wavelength shorter than green wavelengths. For example, a light-emitting material comprising polyfluorene, which is a conjugated polymer, and a triplet light-emitting compound has low luminous efficiency (PATENT DOCUMENT 2 and NON-PATENT DOCUMENT 1).
PATENT DOCUMENT 1: JP-A-2002-50483
PATENT DOCUMENT 2: JP-A-2002-241455
NON-PATENT DOCUMENT 1: APPLIED PHYSICS LETTERS, 80, 13, 2308 (2002)

### DISCLOSURE OF THE INVENTION

### PROBLEMS TO BE SOLVED BY THE INVENTION

Thus, an object of the present invention is to provide a light-emitting material that is excellent in luminous efficiency when used in the preparation of light-emitting devices or the like.

### MEANS FOR SOLVING THE PROBLEMS

The present inventors have conducted diligent studies and consequently completed the present invention by finding that a composition comprising: a compound having a pyrazine ring structure; and a phosphorescence-emitting compound solves the problems described above.
Namely, in a first aspect, the present invention provides a composition comprising: a compound having a pyrazine ring structure; and a phosphorescence-emitting compound.
In a second aspect, the present invention provides a polymer having a residue of the phosphorescence-emitting compound and the pyrazine ring structure.
In a third aspect, the present invention provides a light-emitting thin film, an organic semiconductor thin film and a light-emitting device comprising the composition or the polymer.
In a fourth aspect, the present invention provides a planar light source, a segment display device, a dot matrix display device comprising the light-emitting device and illumination device comprising the light-emitting device as well as a liquid-crystal display device comprising the light-emitting device as a backlight.

### ADVANTAGES OF THE INVENTION

A composition etc. of the present invention has higher luminous efficiency. Thus, the composition etc. of the present invention gives light-emitting devices excellent in luminous efficiency, when used in the preparation of light-emitting devices or the like. Moreover, the composition etc. of the present invention has relatively good light-emitting properties in a short-wavelength emission such as green or blue light emission. This is probably due to relatively easy electron injection and a large lowest triplet excitation energy attributed to the low LUMO of a polymer of the present invention as a compound contained in the composition of the present invention.

BEST MODE FOR CARRYING OUT THE INVENTION

Hereinafter, the present invention will be described in detail.

<Composition>

A composition of the present invention comprises a compound having a pyrazine ring structure and a phosphorescence-emitting compound. The pyrazine ring structure means a group derived by removal of 1 to 4 hydrogen atoms from pyrazine which may have a substituent.
Examples of the pyrazine ring structure include structures represented by the general formulas (1) to (3) and (5) to (7) shown below and structures represented by the formulas (A-1) and (A-2) described later.
When the compound having a pyrazine ring structure is a polymer having a pyrazine ring structure represented by any of the following general formulas (1) to (3) and (5) to (7):

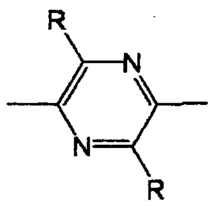
(1)

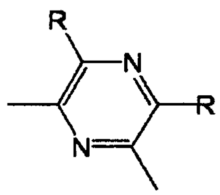
(2)

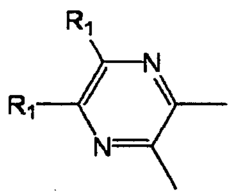
(3)

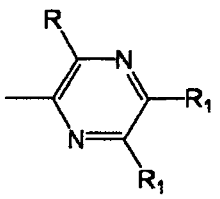
(5)

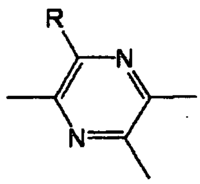
(6)

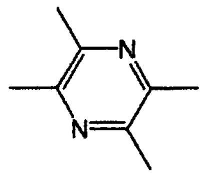
(7)

wherein R and $R_1$ each independently represent a hydrogen atom or a monovalent group, and R's or $R_1$'s may be the same or different,
it is preferred that the polymer compound should have the pyrazine ring structure in the main chain and/or the side chain. In this context, the pyrazine ring structure contained in one molecule is of at least one type.
In the formulas (1) to (3), (5) and (6), R and $R_1$ each independently represent a hydrogen atom or a monovalent group. Preferably, at least one of R's (or $R_1$'s) is a monovalent group. More preferably, all of R's (or $R_1$'s) are a monovalent group.
The monovalent group; examples of which include a halogen atom, an alkyl group, an alkyloxy group, an alkylthio group, an aryl group which may have a substituent, an aryloxy group, an arylthio group, an arylalkyl group, an arylalkyloxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group which may have a substituent, a heteroaryloxy group, a heteroarylthio group, an arylalkenyl group, an arylethynyl group, a substituted carboxyl group and a cyano group; is preferably, an alkyl group, an alkoxy group, an aryl group which may have a substituent or a monovalent heterocyclic group which may have a substituent. In this context, N-valent heterocyclic group (N is 1 or 2) refers to a group derived from a heterocyclic compound by removal of N hydrogen atom(s). In this context, the monovalent heterocyclic group is preferably a monovalent aromatic heterocyclic group.
In a partial structure in which the pyrazine ring structure represented by the formulas (1) to (3), (5) and (6) is attached at a bonding site thereof to a ring structure ,such as an aryl group which may have a substituent, a monovalent heterocyclic group which may have a substituent, an arylene group which may have a substituent or a divalent heterocyclic group which may have a substituent, at least one of the R(s) in the formulas (1) to (3), (5) and (6) is a monovalent group

having preferably three or more atoms in total excluding hydrogen atoms, more preferably five or more atoms in total, even more preferably seven or more atoms in total, particularly preferably ten or more atoms in total.

Moreover, it is also preferred that at least one of R and $R_1$ should be an alkyl group, an alkoxy group, a phenyl group which may have a substituent or a heteroaryl group which may have a substituent. It is also preferred that at least one of R should be an alkyl group, an alkoxy group, a phenyl group which may have a substituent or a heteroaryl group which may have a substituent.

Examples of the compound having a pyrazine ring structure also include a compound having a residue of a compound represented by the following general formula (A-1) or (A-2):

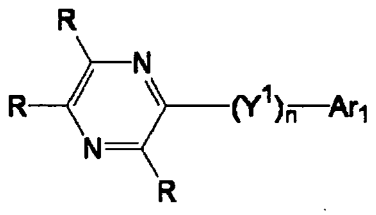

(A-1)

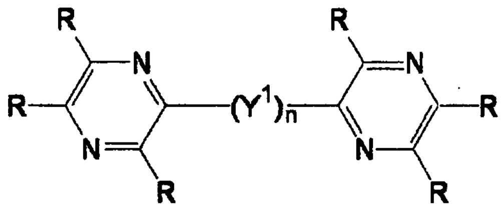

(A-2)

wherein $Y^1$ represents $-C(R^a)(R^b)-$, $-C(=O)-$, $-N(R^c)-$, -O-, $-Si(R^d)(R^e)-$, $-P(R^f)-$, -S- or $-S(=O)_2-$; n is an integer of 0 to 5; $Ar_1$ represents a monovalent aryl group which may have a substituent or a monovalent heterocyclic group which may have a substituent; when a plurality of $Y^1$'s are present, they may be the same or different; $R^a$ to $R^f$ each independently represent a hydrogen atom or a monovalent group; and R is as defined above, and R's may be the same or different. In this context, the pyrazine ring structure contained in one molecule is at least one.

Examples of the monovalent group represented by $R^a$ to $R^f$ include an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a silyloxy group, a substituted silyloxy group, a monovalent heterocyclic group and a halogen atom.

In this context, it is preferred from the viewpoint of luminous efficiency that the compound having a pyrazine ring structure should have a pyrazine ring structure other than a residue of a compound represented by the following general formula (A-3):

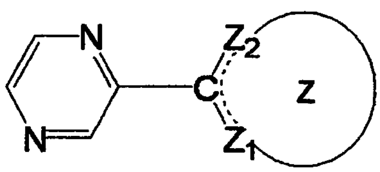

(A-3)

wherein the Z ring is a cyclic structure containing a carbon atom, $Z_1$ and $Z_2$; and $Z_1$ and $Z_2$ each independently represent -C(H)= or -N=.

In the formula (A-3), examples of the cyclic structure include an aromatic ring which may have a substituent and a non-aromatic ring which may have a substituent and specifically include a benzene ring, a heterocyclic ring, an alicyclic hydrocarbon ring, rings made of a plurality of such rings condensed together, and these rings whose hydrogen atoms are partially substituted.

The residue of the compound represented by the formulas (A-1) to (A-3) means a group derived from the compound by removal of some or all of hydrogen atoms.

The compound having a pyrazine ring structure may comprise an additional partial structure. The additional partial structure varies in type depending on whether it is present at the end or not. When the additional partial structure is present at the end, the additional partial structure is a stable monovalent group and is preferably the monovalent substituent or the hydrogen atom included in R and $R_1$ from the viewpoint of easy synthesis and so on. When the additional partial structure is not present at the end, the additional partial structure is a stable multivalent group and is preferably a multivalent group having conjugating properties in terms of a LUMO energy level. Examples of such a group specifically include divalent and trivalent aromatic groups. In this context, the aromatic group refers to a group derived from an organic compound that exhibits aromaticity. Examples of such an aromatic group include phenyl, naphthyl, anthracenyl, pyridyl, quinolyl and isoquinolyl groups.

When the compound having a pyrazine ring structure is a polymer, one preferable example of the additional partial structure which may be contained in the compound includes a structure represented by the following formula (4):

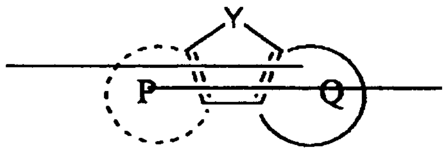

(4)

The structure represented by the formula (4) may have a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group.

In the formula (4), the P ring, which may be present or absent, and the Q ring each independently represent an aromatic ring, wherein two bonds are each present on the P ring or the Q ring in the presence of the P ring and are each present on a Y-containing five-membered or six-membered ring or the Q ring in the absence of the P ring. Moreover, the P ring, the Q ring and the Y-containing five-membered or six-membered ring may have, on the ring, a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group. Y represents -O-, -S-, -Se-, -B($R_1$)-, - Si($R_2$)($R_3$)-, -P($R_4$)-. -P$R_5$ (=O) -, -C($R_6$)($R_7$)-, -N($R_8$)-, C($R_9$) ($R_{10}$) -C ($R_{11}$) ($R_{12}$)-, -O-C($R_{13}$) ($R_{14}$)-, -S-C($R_{15}$) ($R_{16}$)-, - N-C($R_{17}$)($R_{18}$)-, -Si($R_{19}$) ($R_{20}$) -C ($R_{21}$) ($R_{22}$)-, -Si($R_{23}$)($R_{24}$)-Si($R_{25}$($R_{26}$)-, -C($R_{27}$)=C($R_{28}$)-, -N=C ($R_{29}$) - or - Si($R_{30}$)=C($R_{31}$)-. In this context, $R_1$ to $R_{31}$ each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a silyloxy group, a substituted silyloxy group, a monovalent heterocyclic group or a halogen atom. Among them, an alkyl group, an alkoxy group, an alkylthio group, an aryl group, an aryloxy group, an arylthio group, an arylalkyl group, an arylalkoxy group, and a monovalent heterocyclic group are preferable. An alkyl group, an alkoxy group, an aryl group and a monovalent heterocyclic group are more preferable, and an alkyl group and an aryl group are particularly preferable.

Examples of the structure represented by the formula (4) include: a structure represented by the following formula (4-1), (4-2) or (4-3):

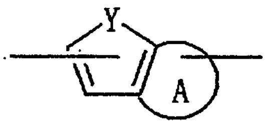

(4-1)

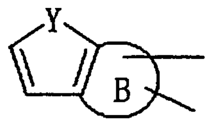

(4-2)

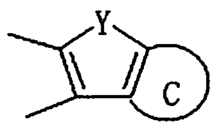

(4-3)

wherein the A ring, the B ring and the C ring each independently represent an aromatic ring; the formulas (4-1), (4-2)

and (4-3) may each have a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group; and Y is as defined above; and a structure represented by the following formula (4-4) or (4-5):

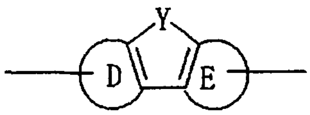

(4-4)

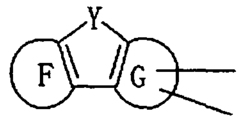

(4-5)

wherein the D ring, the E ring, the F ring and the G ring each independently represent an aromatic ring which may have a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group; and Y is as defined above.
In the formulas (4-4) and (4-5), Y is preferably a carbon atom, a nitrogen atom, an oxygen atom or a sulfur atom, because higher luminous efficiency can be obtained.

In the formulas (4-1), (4-2), (4-3), (4-4) and (4-5), the A ring, the B ring, the C ring, the D ring, the E ring, the F ring and the G ring each independently represent an aromatic ring. Examples of the aromatic ring include: aromatic hydrocarbon rings, such as benzene, naphthalene, anthracene, tetracene, pentacene, pyrene and phenanthrene rings; and heterocyclic aromatic rings, such as pyridine, bipyridine, phenanthroline, quinoline, isoquinoline, thiophene, furan and pyrrole rings.

When the compound having a pyrazine ring structure is a polymer, one preferable example of the additional partial structure which may be contained in the compound includes a group represented by the following formula:

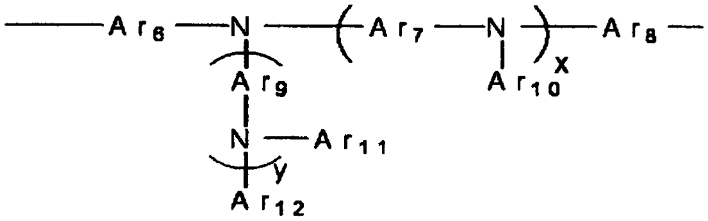

wherein $Ar_6$, $Ar_7$, $Ar_8$ and $Ar_9$ each independently represent an arylene group or a divalent heterocyclic group; $Ar_{10}$, $Ar_{11}$ and $Ar_{12}$ each independently represent an aryl group or a monovalent heterocyclic group; $Ar_6$, $Ar_7$, $Ar_8$, $Ar_9$ and $Ar_{10}$ may have a substituent; and x and y are each independently 0 or 1, wherein $0 \leq x+y \leq 1$.

In the present invention, the "polymer" means a compound containing at least two or more of the same structural units (repeating units). When the compound having a pyrazine ring structure is a polymer, the compound has a polystyrene equivalent weight-average molecular weight of preferably $3\times10^2$ or larger, more preferably $3\times10^2$ to $1\times10^7$, even more preferably $1\times10^3$ to $1\times10^7$, particularly preferably $1\times10^4$ to $1\times10^7$ from the viewpoint of film formability.

The compound having a pyrazine ring structure can be used in a wide emission wavelength region. The compound has a lowest triplet excitation energy (hereinafter, also referred to as a "$T_1$ energy") of preferably 2.7 eV or larger, more preferably 2.8 eV or larger, even more preferably 2.9 eV or larger, still preferably 3.0 eV or larger, particularly preferably 3.1 eV or larger. Moreover, the upper limit thereof is usually 5.0 eV.

The compound having a pyrazine ring structure has an absolute value of the lowest unoccupied molecular orbital (LUMO) energy level of preferably 2.1 eV or larger, more preferably 2.2 eV or larger, even more preferably 2.3 eV or larger, particularly preferably 2.4 eV or larger. Moreover, the upper limit thereof is usually 4.0 eV.

In the present specification, the values of the $T_1$ energy and the LUMO energy level are values calculated by

a computational chemical approach. In the present specification, the quantum chemical calculation program Gaussian 03 is used as the computational chemical approach. The structure of the ground state was optimized by an HF (Hartree-Fock) method, and the values of the $T_1$ energy and the LUMO energy level were calculated in the optimized structure using a time-dependent density functional method at a B3P86 level. In this procedure, 6-31g* was used as a basis function.

When the compound having a pyrazine ring structure is a polymer and comprises one type of repeating unit which is defined as A, the compound is represented by the following formula:

$$-\left( A \right)_n-$$

wherein n represents the degree of polymerization.

In this context, a LUMO energy level is calculated for structures wherein n=1, 2 and 3, and a value at n=∞ in linear approximation with the calculated LUMO energy levels as functions of (1/n) is defined as the LUMO energy level of the compound. The $T_1$ energy is defined in the same way as above.

When the compound having a pyrazine ring structure is a polymer and comprises a plurality of repeating units, the lowest $T_1$ energy among all combinations present is defined as the $T_1$ energy of the compound. The LUMO energy level of a structure that gives the lowest $T_1$ energy is defined as the LUMO energy level of the compound.

When the compound having a pyrazine ring structure comprises a pyrazine ring structure represented by the general formula (1), (2), (3), (5), (6) or (7), it is preferred that the compound should have a partial structure which is adjacent to the pyrazine ring structure and which has at least two π-conjugated electrons. The pyrazine ring structure represented by the general formula (1), (2), (3), (5), (6) or (7) and the (additional) partial structure which is adjacent to the pyrazine ring structure and which has at least two π-conjugated electrons, usually form therebetween a dihedral angle of 20° or larger, preferably 30° or larger, more preferably 40° or larger, even more preferably 50° or larger, still preferably 60° or larger, particularly preferably 70° or larger.

In this context, the dihedral angle in the present invention means the degree of an angle calculated from the optimized structure of the ground state. The dihedral angle is defined by, in the pyrazine ring structure represented by the general formula (1), (2), (3), (5), (6) or (7), a carbon atom ($a_1$) at the bonding site with the partial structure having at least two π-conjugated electrons and a carbon atom ($a_2$) adjacent thereto, and by, in the structure bonded to the pyrazine ring structure, an atom ($a_3$) at the position of the bond and an atom ($a_4$) adjacent thereto.

In this context, when a plurality of atom combinations ($a_1$, $a_2$, $a_3$ and $a_4$) can be selected, a dihedral angle is calculated in all the cases, and the lowest value among all absolute values is defined as the dihedral angle. The atoms ($a_3$) and ($a_4$) may be an atom with or without a π-conjugated electron and are preferably an atom with a π-conjugated electron, more preferably a carbon atom, a nitrogen atom, a silicon atom or a phosphorus atom. In the present specification, it is calculated from the optimized structure of the ground state of the structure determined by the computational chemical approach (i.e., a structure that offers the lowest heat of formation of the structure).

Preferable examples of the compound having a pyrazine ring structure include polymers comprising, as a repeating unit, a structure represented by the general formula (1), (2), (3), (5), (6) or (7), and polymers comprising a structure represented by the general formula (1), (2), (3), (5), (6) or (7) as well as any structure selected from an aromatic ring, a heteroatom-containing five- or more membered heterocyclic ring, aromatic amine and a structure represented by the general formula (4). Moreover, examples of the compound having a pyrazine ring structure include polymers represented by the formulas (5-1) to (5-26) shown below.

In the formulas (5-1) to (5-26) shown below, R represents a hydrogen atom or a substituent. The substituent represented by R is exemplified by a halogen atom, an alkyl group, an alkyloxy group, an alkylthio group, an aryl group, an aryloxy group, an arylthio group, an arylalkyl group, an arylalkyloxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylethynyl group, a substituted carboxyl group and a cyano group. A plurality of R's may be the same or different. R is more preferably an alkyl group, an aryl group, an arylalkyl group or a monovalent heterocyclic group.

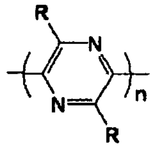
(5-1)

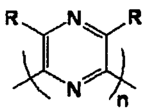
(5-2)

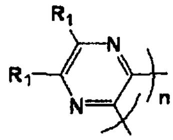
(5-3)

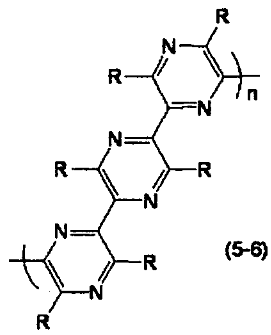
(5-6)

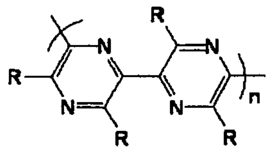
(5-4)

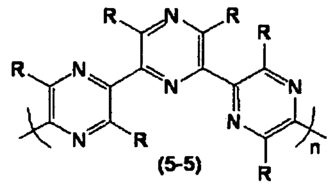
(5-5)

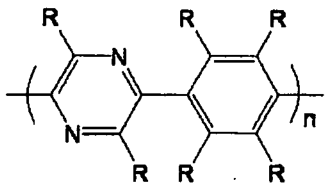
(5-7)

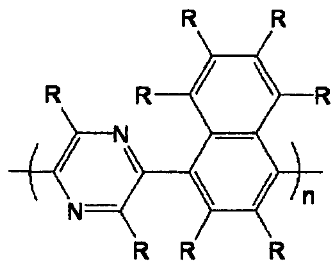
(5-8)

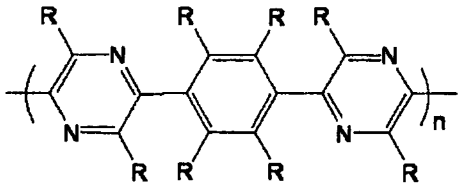
(5-9)

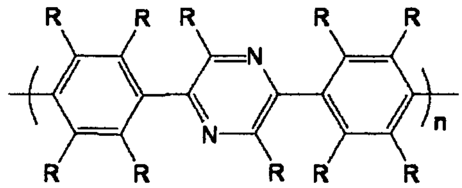
(5-10)

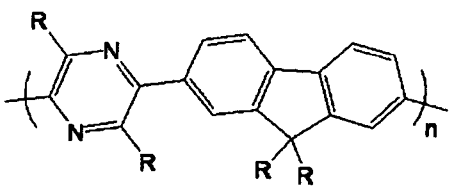
(5-11)

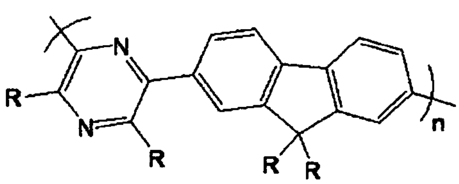
(5-12)

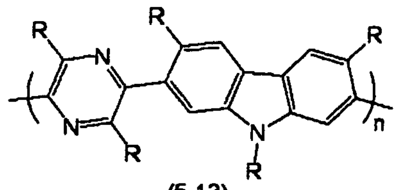
(5-13)

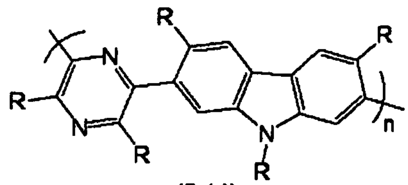
(5-14)

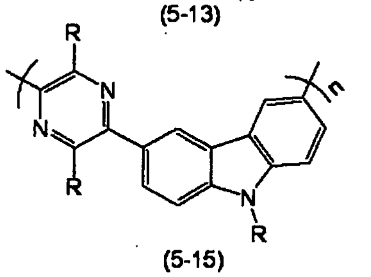
(5-15)

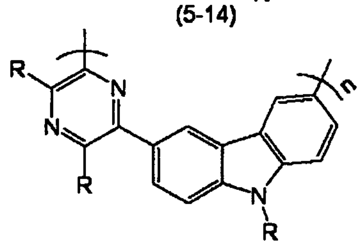
(5-16)

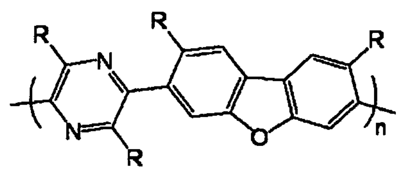
(5-17)

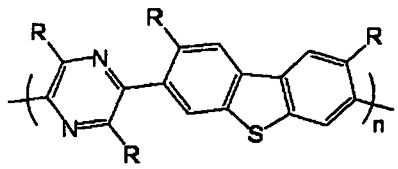
(5-18)

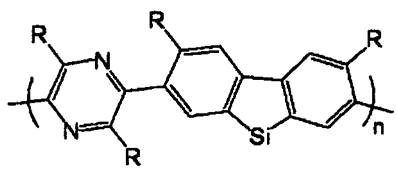
(5-19)

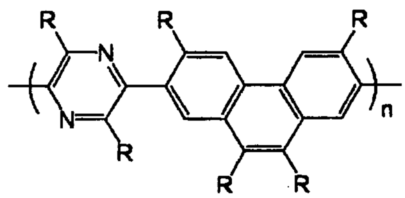
(5-20)

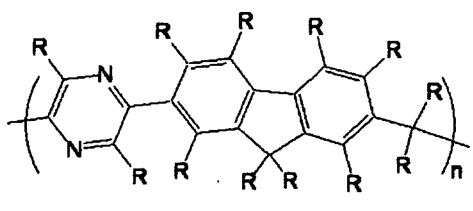
(5-21)

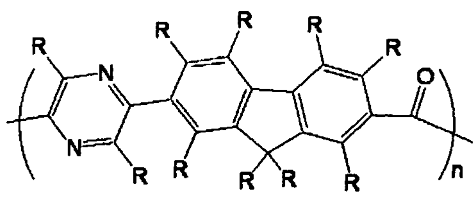
(5-22)

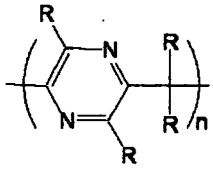

(5-23)

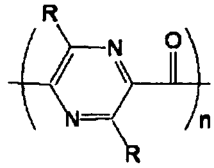

(5-24)

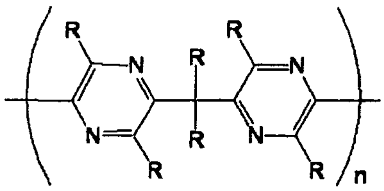

(5-25)

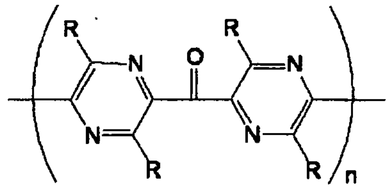

(5-26)

wherein n represents the degree of polymerization; and R is as defined above, and a plurality of R's may be the same or different.

Moreover, examples of the specific structure of the compound having a pyrazine ring structure include the following polymers:

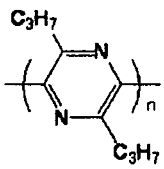

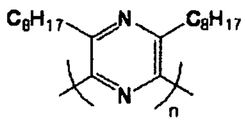

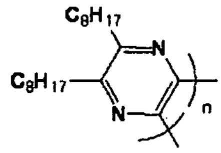

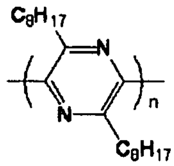

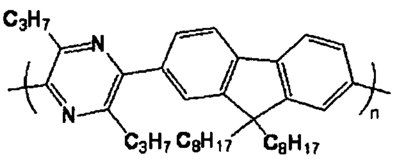

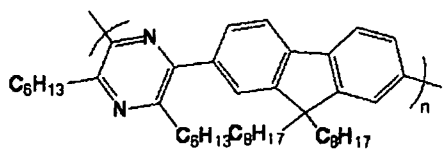

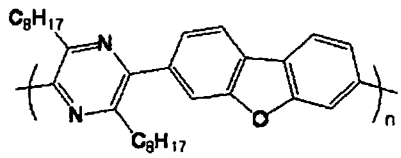
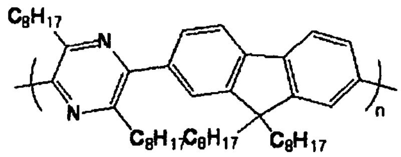
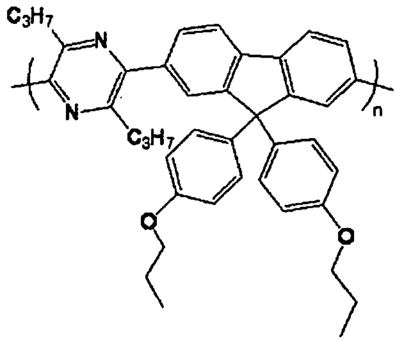
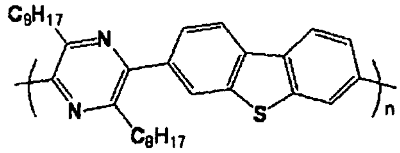
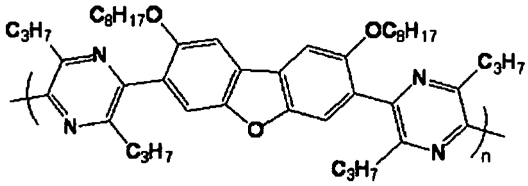
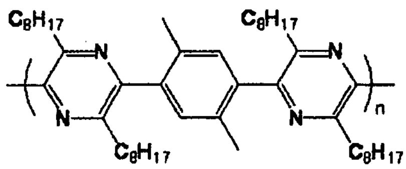
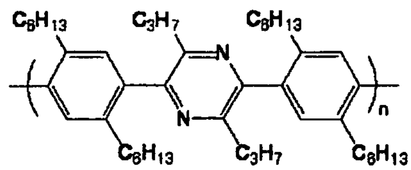
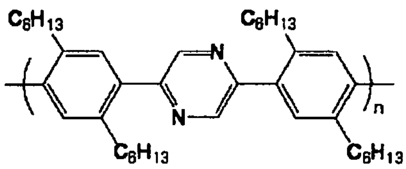
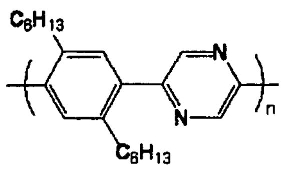
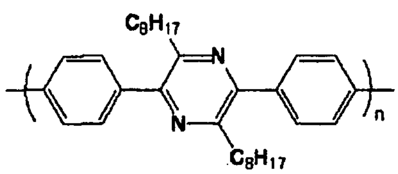

wherein n represents the degree of polymerization.

Examples of the compound having a pyrazine ring structure can also include the following compounds:

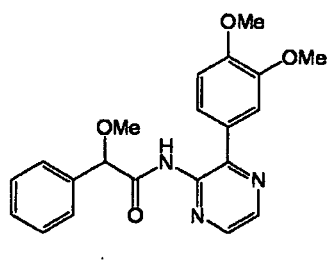
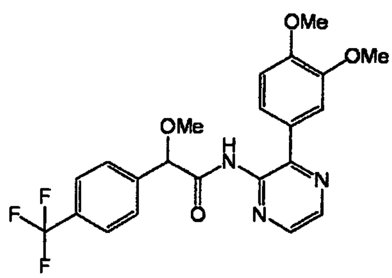

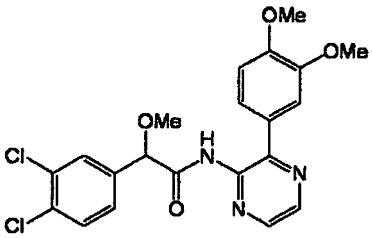

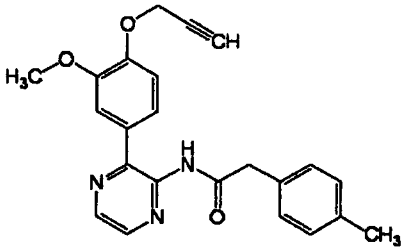

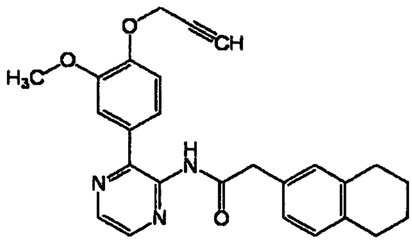

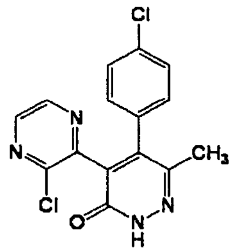

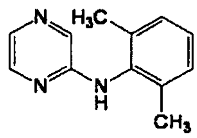

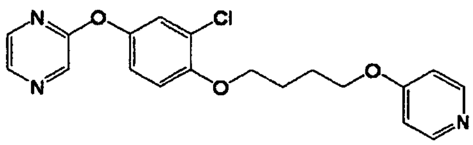

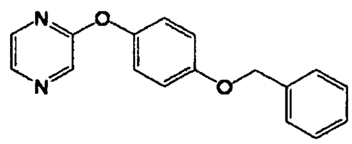

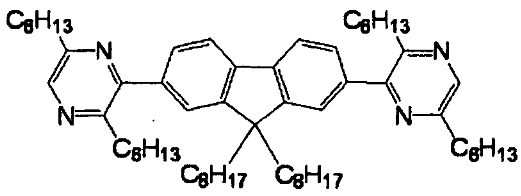

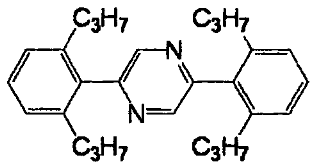

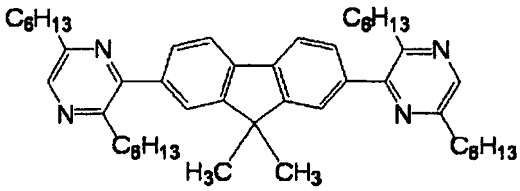

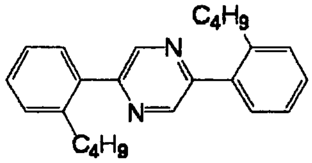

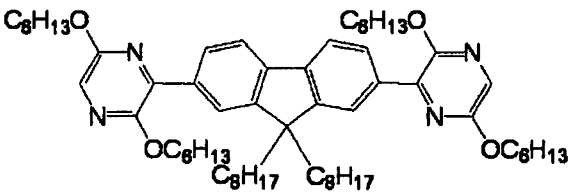

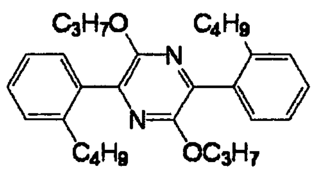

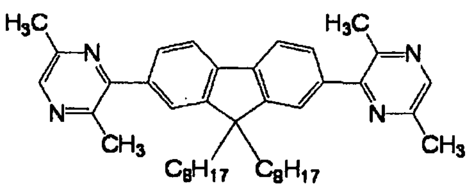

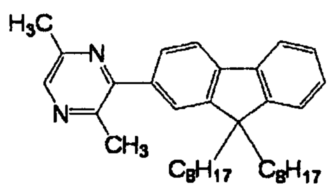

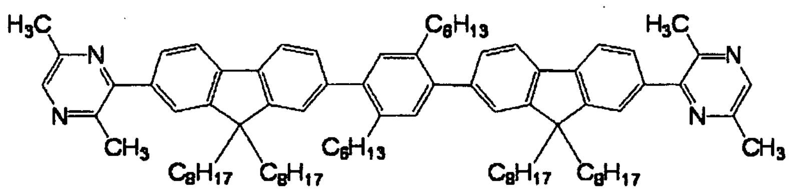

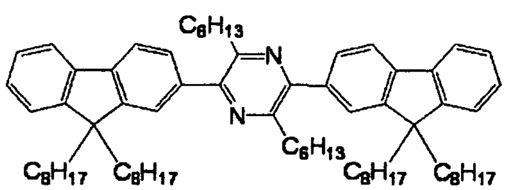

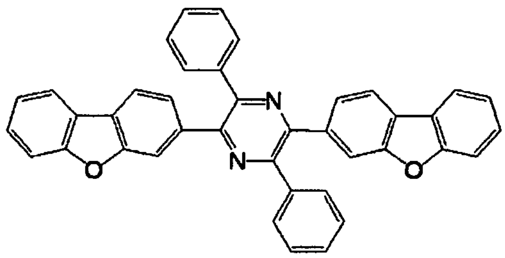

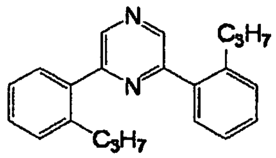

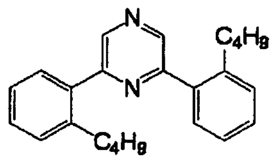

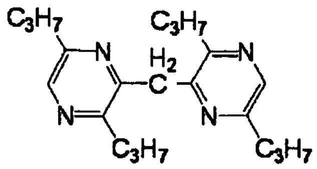

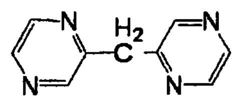

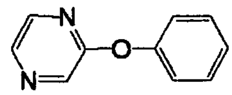

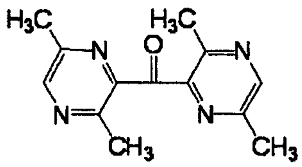

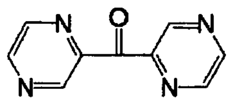

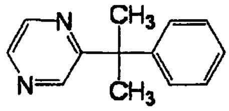

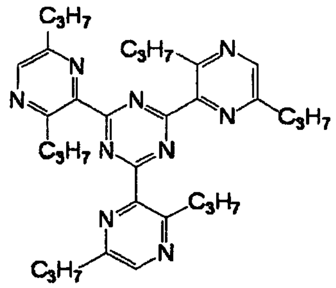

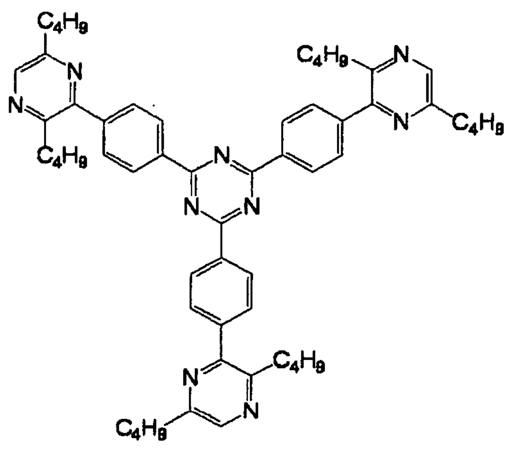

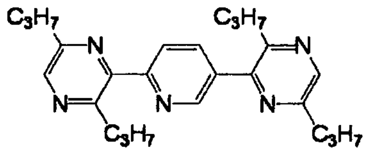

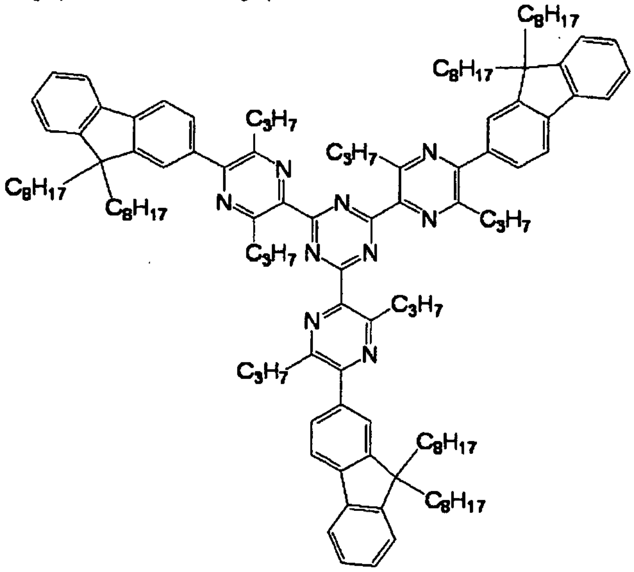

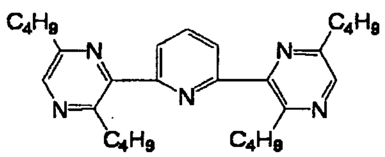

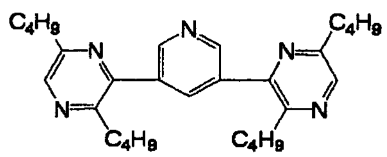

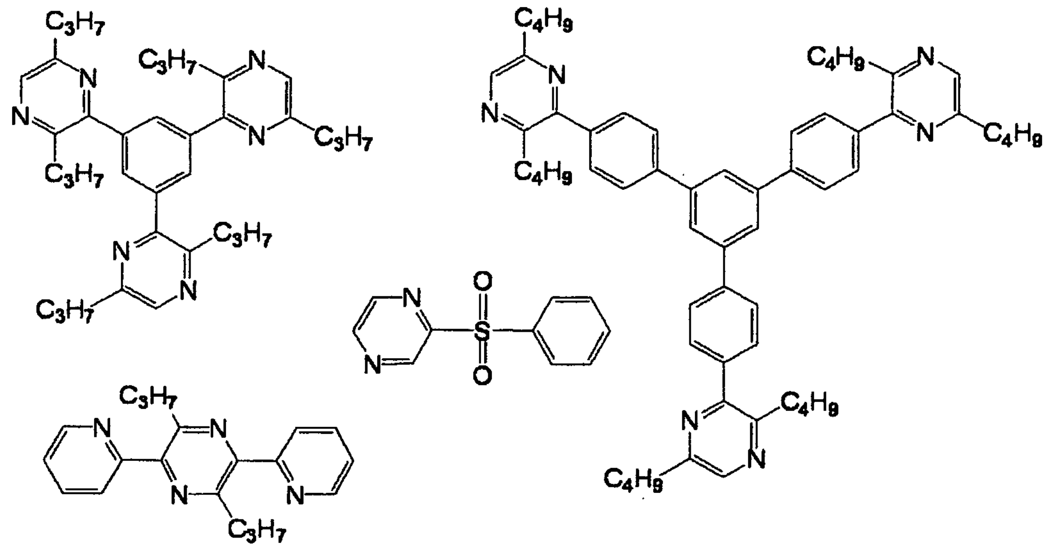

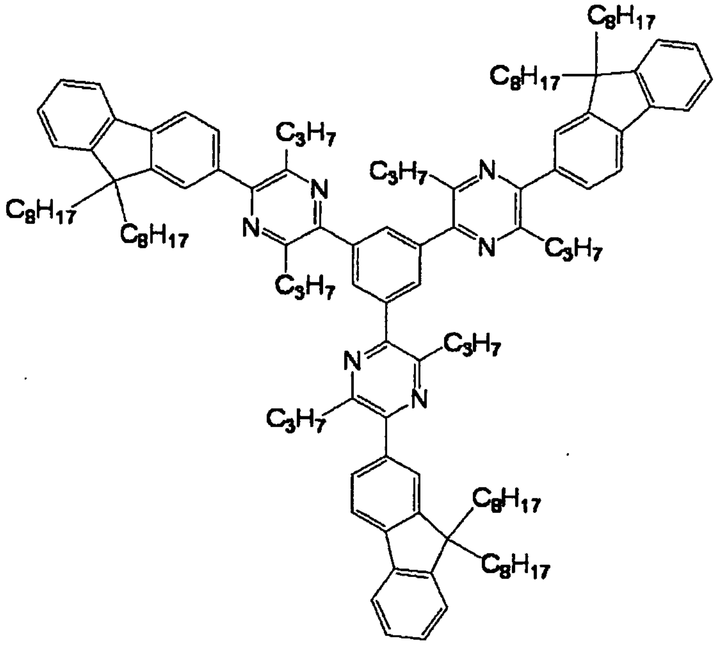

Compounds known in the art, such as triplet light-emitting complexes, can be used as the phosphorescence-emitting compound. Examples thereof include those previously used as low-molecular EL-emitting materials. They are disclosed in, e.g., Nature, (1998), 395, 151; Appl. Phys, Lett. (1999), 75 (1), 4; Proc. SPIE-Int. Soc. Opt. Eng. (2001), 4105 (Organic Light-Emitting Materials and Devices IV), 119; J. Am. Chem. Soc., (2001), 123, 4304; Appl. Phys. Lett., (1997), 71 (18), 2596; Syn. Met., (1998), 94 (1), 103; Syn. Met., (1999), 99 (2), 1361; Adv. Mater., (1999), 11 (10), 852; Inorg. Chem., (2003), 42, 8609; Inorg. Chem., (2004), 43, 6513; Journal of the SID 11/1, 161 (2003); WO2002/066552; WO2004/020504; and WO2004/020448. It is particularly preferred from the viewpoint of obtaining high luminous efficiency that in the highest occupied molecular orbital (HOMO) of a metal complex, the sum of the square of orbital coefficients of outermost d orbitals of the central metal should occupy 1/3 or larger of the sum of the square of all the atomic orbital coefficients. Examples thereof include ortho-metalated complexes having a transition metal belonging to the 6th period as a central metal.

The central metal in the triplet light-emitting complexes is usually an atom with an atomic number of 50 or higher and is a metal that is capable of causing spin-orbit interaction in the complex and causing intersystem crossing between singlet and triplet states. Examples thereof include gold, platinum, iridium, osmium, rhenium, tungsten, europium, terbium, thulium, dysprosium, samarium, praseodymium, gadolinium and ytterbium atoms. The central metal is preferably a gold, platinum, iridium, osmium, rhenium or tungsten atom, more preferably a gold, platinum, iridium, osmium or

rhenium atom, even more preferably a gold, platinum, iridium or rhenium atom, particularly preferably a platinum or iridium atom.

Examples of a ligand in the triplet light-emitting complex include 8-quinolinol and derivatives thereof, benzoquinolinol and derivatives thereof and 2-phenyl-pyridine and derivatives thereof.

The phosphorescence-emitting compound is preferably a compound having a substituent, such as an alkyl group, an alkoxy group, an aryl group which may have a substituent or a heteroaryl group which may have a substituent from the viewpoint of solubility. Furthermore, the substituent has preferably three or more atoms in total excluding hydrogen atoms, more preferably five or more atoms in total, even more preferably seven or more atoms in total, particularly preferably ten or more atoms in total. Moreover, it is preferred that at least one or more of the substituents should be present in each ligand. The types of the substituents may be the same or different in each ligand.

Examples of the phosphorescence-emitting compound include the followings:

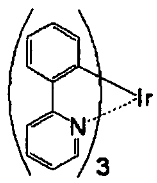
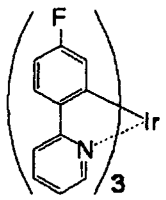
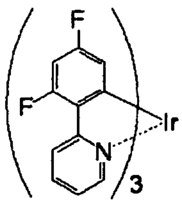
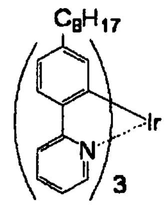
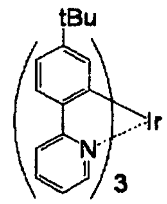

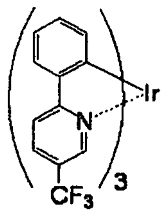
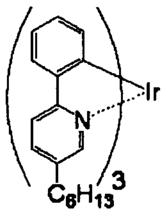
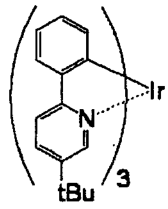
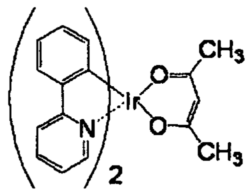

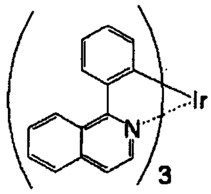
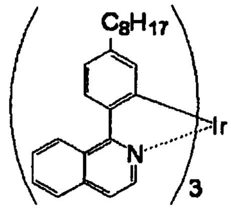
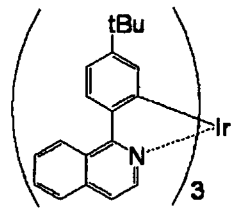
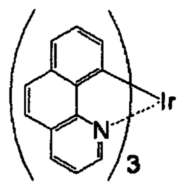

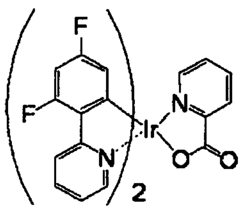
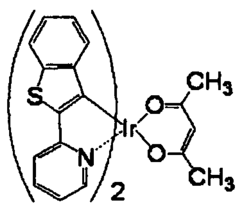
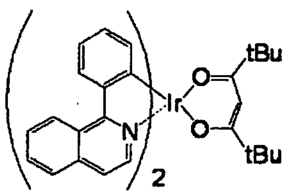

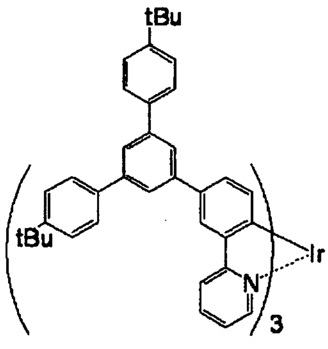 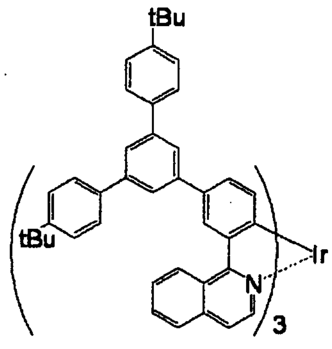 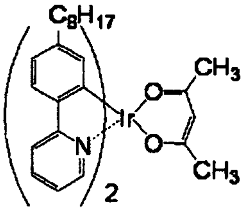

The amount of the phosphorescence-emitting compound in the composition of the present invention is usually 0.01 to 80 parts by weight, preferably 0.1 to 30 parts by weight, more preferably 0.1 to 15 parts by weight, particularly 0.1 to 10 parts by weight, with respect to 100 parts by weight of the compound having a pyrazine ring structure. In this context, in the composition of the present invention, the compound having a pyrazine ring structure and the phosphorescence-emitting compound are each used alone or in combination of two or more thereof.

The composition of the present invention may comprise an optional component, to the extent that the object of the present invention is not impaired by it, in addition to the compound having a pyrazine ring structure and the phosphorescence-emitting compound. Examples of this optional component include a hole transport material, an electron transport material and an antioxidant.

Examples of the hole transport material include aromatic amine, carbazole derivatives and polyparaphenylene derivatives previously known in the art as hole transport materials for organic EL devices.

Examples of the electron transport material include materials previously known in the art as electron transport materials for organic EL devices, such as oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives and metal complexes of 8-hydroxyquinoline and derivatives thereof.

In the composition of the present invention, it is preferred from the viewpoint of high luminous efficiency that the lowest triplet excitation energy (ETP) of the polymer or the compound having a pyrazine ring structure should satisfy, with a lowest triplet excitation energy (ETT) of the phosphorescence-emitting compound, the following formula:

$$ETT>ETP-0.20\ (eV).$$

A light-emitting thin film of the present invention is obtained by forming a thin film comprising the composition of the present invention. Examples of the method for forming a thin film include solution application, deposition and transfer. In the solution application, application methods can be used, such as spin coating, casting, micro-gravure coating, gravure coating, bar coating, roll coating, wire bar coating, dip coating, spray coating, screen printing, flexographic printing, offset printing and inkjet printing methods.

A solvent that can dissolve or uniformly disperse the composition therein is preferable. The solvent is exemplified by chlorine solvents (chloroform, methylene chloride, 1,2-dichloroethane, 1,1,2-trichloroethane, chlorobenzene, O-dichlorobenzene, etc.), ether solvents (tetrahydrofuran, dioxane, etc.), aromatic hydrocarbon solvents (toluene, xylene, etc.), aliphatic hydrocarbon solvents (cyclohexane, methylcyclohexane, n-pentane, n-hexane, n-heptane, n-octane, n-nonane, n-decane, etc.), ketone solvents (acetone, methyl ethyl ketone, cyclohexanone, etc.), ester solvents (ethyl acetate, butyl acetate, ethyl cellosolve acetate, etc.), polyhydric alcohols and derivatives thereof (ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexanediol, etc.), alcoholic solvents (methanol, ethanol, propanol, isopropanol, cyclohexanol, etc.), sulfoxide solvents (dimethyl sulfoxide, etc.) and amide solvents (N-methyl-2-pyrrolidone, N,N-dimethylformamide, etc.). The solvent can be selected from among them and used. Moreover, these organic solvents may be used alone or in combination of two or more thereof.

When an inkjet printing method is used, methods known in the art for selecting a solvent in the solution or additives can be used for improving discharge from a head, nonuniformity, and so on. In this case, it is preferred that the solution should have a viscosity of 1 to 100 mPa·s at 25°C. Moreover, excessive vaporization tends to make repetitive discharge from a head difficult. From these points of view, examples of preferable solvents used include single or mixed

solvents containing anisole, bicyclohexyl, xylene, tetralin and dodecylbenzene. In general, a solution for inkjet suitable for the composition used can be obtained by a method comprising mixing a plurality of solvents, a method comprising adjusting a concentration in the solution of the composition, and so on.

<Polymer>

A polymer of the present invention has a residue of the phosphorescence-emitting compound and the pyrazine ring structure. This pyrazine ring structure is the same as those described and exemplified in the paragraph of the composition. Examples of the polymer of the present invention include (1) a polymer having the structure of the phosphorescence-emitting compound in the main chain of the polymer, (2) a polymer having the structure of the phosphorescence-emitting compound in the end of the polymer, and (3) a polymer having the structure of the phosphorescence-emitting compound in the side chain of the polymer.

<Light-emitting device>

Next, a light-emitting device of the present invention will be described.
The light-emitting device of the present invention is obtained by using the composition or the polymer. The light-emitting device usually comprises the composition or the polymer at least at a certain site between electrodes comprising an anode and a cathode and, preferably, comprises them in the form of the light-emitting thin film as a light-emitting layer. Moreover, the light-emitting device may comprise one or more of layers known in the art which have other functions from the viewpoint of improving performance, such as luminous efficiency and durability. Examples of such layers include a charge transport layer (i.e., a hole transport layer and an electron transport layer), a charge block layer (i.e., a hole block layer and an electron block layer), a charge injection layer (i.e., a hole injection layer and an electron injection layer) and a buffer layer. In this context, in the light-emitting device of the present invention, the light-emitting layer, the charge transport layer, the charge block layer, the charge injection layer, the buffer layer, and the like may each be single-layered or double- or more layered.
The light-emitting layer is a layer having the function of emitting light. The hole transport layer is a layer having the function of transporting holes. The electron transport layer is a layer having the function of transporting electrons. These electron transport and hole transport layers are collectively referred to as a charge transport layer. Moreover, the charge block layers are layers having the function of trapping holes or electrons into the light-emitting layer, among which a layer for transporting electrons and blocking holes is reffered to as a hole block layer and a layer for transporting holes and blocking electrons is reffered to as an electron block layer.
Examples of the buffer layer include a layer which is located adjacently to the anode and comprises a conductive polymer.
Specific examples of the light-emitting devices of the present invention include the following structures a) to q):

a) anode/light-emitting layer/cathode;
b) anode/hole transport layer/light-emitting layer/cathode;
c) anode/light-emitting layer/electron transport layer/cathode;
d) anode/light-emitting layer/hole block layer/cathode;
e) anode/hole transport layer/light-emitting layer/electron transport layer/cathode;
f) anode/charge injection layer/light-emitting layer/cathode;
g) anode/light-emitting layer/charge injection layer/cathode;
h) anode/charge injection layer/light-emitting layer/charge injection layer/cathode;
i) anode/charge injection layer/hole transport layer/light-emitting layer/cathode;
j) anode/hole transport layer/light-emitting layer/charge injection layer/cathode;
k) anode/charge injection layer/hole transport layer/light-emitting layer/charge injection layer/cathode;
l) anode/charge injection layer/light-emitting layer/electron transport layer/cathode;
m) anode/light-emitting layer/electron transport layer/charge injection layer/cathode;
n) anode/charge injection layer/light-emitting layer/electron transport layer/charge injection layer/cathode;
o) anode/charge injection layer/hole transport layer/light-emitting layer/electron transport layer/cathode;
p) anode/hole transport layer/light-emitting layer/electron transport layer/charge injection layer/cathode; and
q) anode/charge injection layer/hole transport layer/light-emitting layer/electron transport layer/charge injection layer/cathode.

In this context, the "/"represents that the layers are stacked adjacently. The same shall apply hereinafter. In this context, the light-emitting layer, the hole transport layer and the electron transport layer may each independently be double- or more layered for use.

When the light-emitting devices of the present invention has a hole transport layer (usually, the hole transport layer contains a hole transport material), materials known in the art can be selected appropriately and used as the hole transport material. Specific examples thereof include polymer hole transport materials, such as polyvinylcarbazole and derivatives thereof, polysilane and derivatives thereof, polysiloxane derivatives having aromatic amine in the side chain or the main chain, pyrazoline derivatives, arylamine derivatives, stilbene derivatives, triphenyldiamine derivatives, polyaniline and derivatives thereof, polythiophene and derivatives thereof, polypyrrole and derivatives thereof, poly(p-phenylene vinylene) and derivatives thereof and poly(2,5-thienylene vinylene) and derivatives thereof. The hole transport material is exemplified by those described in JP-A-63-70257, JP-A-63-175860, JP-A-2-135359, JP-A-2-135361, JP-A-2-209988, JP-A-3-37992 and JP-A-3-152184.

When the light-emitting device of the present invention has an electron transport layer (usually, the electron transport layer contains an electron transport material), materials known in the art can be selected appropriately and used as the electron transport material. Specific examples thereof include oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, metal complexes of 8-hydroxyquinoline and derivatives thereof, polyquinoline and derivatives thereof, polyquinoxaline and derivatives thereof and polyfluorene and derivatives thereof.

The film thicknesses of the hole transport layer and the electron transport layer differ in optimum value depending on materials used and need only to be selected to offer appropriate values of a drive voltage and luminous efficiency. These layers require at least a thickness that prevents pinhole formation. Too thick a film thickness is not preferable, because a high drive voltage for the device is required. Thus, the hole transport layer and the electron transport layer have a film thickness of, for example, 1 nm to 1 μm, preferably 2 nm to 500 nm, more preferably 5 nm to 200 nm.

Moreover, of the charge transport layers disposed adjacently to the electrode, those having the function of improving charge injection efficiency from the electrode and the effect of decreasing a drive voltage for the device may particularly be called a charge injection layer (i.e., a general term for a hole injection layer and an electron injection layer; the same shall apply hereinafter.

Furthermore, the charge injection layer or an insulating layer (usually, 0.5 nm to 4 nm in average film thickness; the same shall apply hereinafter.) may be disposed adjacently to the electrode for improving adhesion to the electrode and charge injection from the electrode. Moreover, a thin buffer layer may be inserted in the interface of the charge transport layer or the light-emitting layer for, for example, improving adhesion of the interface or preventing contamination.

The order in which layers are stacked, the number of layers, and the thickness of each layer can be selected appropriately in consideration of luminous efficiency and an device lifetime.

Examples of the charge injection layer include: a layer comprising a conductive polymer; a layer which is disposed between the anode and the hole transport layer and comprises a material having an ionization potential intermediate between the corresponding values of the anode material and the hole transport material contained in the hole transport layer; and a layer which is disposed between the cathode and the electron transport layer and comprises a material having electron affinity intermediate between the corresponding values of the cathode material and the electron transport material contained in the electron transport layer.

The material used in the charge injection layer needs only to be selected appropriately according to the relationship with the materials for the electrode or the adjacent layer and is exemplified by polyaniline and derivatives thereof, polythiophene and derivatives thereof, polypyrrole and derivatives thereof, polyphenylene vinylene and derivatives thereof, polythienylene vinylene and derivatives thereof, polyquinoline and derivatives thereof, polyquinoxaline and derivatives thereof, conductive polymers (e.g., polymers containing an aromatic amine structure in the main chain or the side chain), metallophthalocyanine (e.g., copper phthalocyanine) and carbon.

The insulating layer has the function of facilitating charge injection. Examples of materials for the insulating layer include metal fluorides, metal oxides and organic insulating materials. Examples of the light-emitting device comprising the insulating layer include: a light-emitting device comprising the insulating layer disposed adjacently to the cathode; and a light-emitting device comprising the insulating layer disposed adjacently to the anode.

The light-emitting device of the present invention is usually formed on a substrate. The substrate may be any one as long as it is not transferred when the electrode and subsequently the organic layer are formed thereon. Examples thereof include glass, plastic, polymer films and silicon substrates. When an opaque substrate is used, it is preferred that the electrode on the other side should be transparent or semitransparent.

At least one of the anode and the cathode contained in the light-emitting device of the present invention is usually transparent or semitransparent. Particularly, it is preferred that the anode should be transparent or semitransparent.

Conductive metal oxide films, semitransparent metal thin films, or the like are usually used as materials for the anode. A specific example thereof is a film (NESA, etc.) prepared using conductive glass comprising indium oxide, zinc oxide, tin oxide and their complex indium tin oxide (ITO) or indium zinc oxide, and the like. Gold, platinum, silver, copper,

or the like is also used. ITO, indium zinc oxide and tin oxide are preferable. Examples of the preparation method include vacuum deposition, sputtering, ion plating and plating methods. Alternatively, transparent organic conductive films, such as polyaniline and derivatives thereof and polythiophene and derivatives thereof may be used as the anode. In this context, the anode may have a double- or more layered structure.

Materials having a small work function are usually preferable as materials for the cathode. For example, metals, such as lithium, sodium, potassium, rubidium, cesium, beryllium, magnesium, calcium, strontium, barium, aluminum, scandium, vanadium, zinc, yttrium, indium, cerium, samarium, europium, terbium and ytterbium, and alloys of two ore more thereof or alloys of one or more thereof with one or more of gold, silver, platinum, copper, manganese, titanium, cobalt, nickel, tungsten and tin, and graphite or intercalated graphite are used. Examples of the alloys include magnesium-silver, magnesium-indium, magnesium-aluminum, indium-silver, lithium-aluminum, lithium-magnesium, lithium-indium and calcium-aluminum alloys. In this context, the cathode may have a double- or more layered structure.

The light-emitting device of the present invention can be used, for example, in a planar light source and a display device (a segment display device, a dot matrix display device, a liquid-crystal display device, etc.) or as a backlight thereof (i.e., in, e.g., a liquid-crystal display device comprising the' light-emitting device as a backlight).

To obtain planar light emission using the light-emitting device of the present invention, planar anode and cathode need only to be arranged such that they overlap with each other. Moreover, to obtain patterned light emission, methods are used which include: a method comprising placing a mask provided with a window with the pattern on the surface of the planar light-emitting device; a method comprising forming an extremely thick organic layer of a non-light-emitting portion such that the portion becomes substantially non-light-emitting; and a method comprising forming the pattern on either or both of the anode and the cathode. The pattern is formed by any of these methods, and some electrodes are arranged such that they can independently be switched between the ON and OFF positions. As a result, a segment-type display device is obtained which can display numbers, letters, simple symbols, and so on. Furthermore, to obtain a dot matrix element, both the anode and the cathodes need only to be formed in a striped form and arranged orthogonally. Partially colored display or multicolor display is achieved by a method comprising separately applying two or more types of polymer phosphors differing in the color of emitted light or by a method using a color filter or a fluorescence conversion filter. The dot matrix device can be driven passively or may be driven actively in combination with TFT or the like. These display devices can be used as display devices for computers, televisions, mobile terminals, cellular phones, car navigation systems, video camera view finders, and so on. Furthermore, the planar light-emitting device is a self-emitting, thin device and can be used preferably as a planar light source for backlights in liquid-crystal display devices or as a light source for planar illumination. Moreover, the planar light-emitting device can be used as a light source or a display device having a curved surface, when a flexible substrate is used therein.

The composition and the polymer of the present invention are not only useful in the preparation of devices but also can be used as a semiconductor material (e.g., an organic semiconductor material), a light-emitting material, an optical material or a conductive material (e.g., which is applied by doping). Thus, the composition and the polymer can be used to prepare films, such as light-emitting thin films, conductive thin films and organic semiconductor thin films.

The composition and the polymer of the present invention can be used to form conductive thin films and semiconductor thin films and make them into devices in the same way as in the method for preparing a light-emitting thin film used as the light-emitting layer in the light-emitting devices. For the semiconductor thin films, it is preferred that, of electron mobility and hole mobility, the larger one should have $10^{-5}$ $cm^2$/V/second or larger. Moreover, the organic semiconductor thin films can be used in organic solar cells, organic transistors, and so on.

EXAMPLES

Hereinafter, the present invention will be described more specifically with reference to Examples. However, the present invention is not intended to be limited to them.

<Example 1>

A polymer (P-1) represented by the following formula:

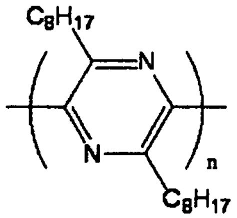

(P-1)

had the lowest triplet excitation energy $T_1$ (l/n=0) of 3.1 eV as an extrapolated value at n=∞ and an absolute value of the lowest unoccupied molecular orbital energy level $E_{LUMO}$ (l/n=0) of 2.3 eV. The pyrazine ring and the partial structure (in the present Example, the pyrazine ring) adjacent to the pyrazine ring formed a dihedral angle of 61° therebetween. The parameter calculation was conducted by a computational chemical approach described in Detailed Description of the Invention. Specifically, the structure was optimized according to an HF method by simplifying the following repeating unit (M-1) in the polymer (P-1) to the structure (M-1a):

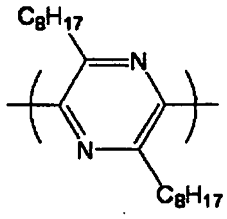

(M-1)

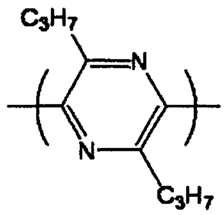

(M-1a)

In this procedure, 6-31G* was used as a basis function. Then, the lowest unoccupied molecular orbital energy level and the lowest triplet excitation energy were calculated by a time-dependent density functional method at a B3P86 level using the same basis. The validity of the simplified chemical structure was confirmed according to a method described in JP-A-2005-126686 based on the small alkyl side chain length dependence of the lowest triplet excitation energy and the lowest unoccupied molecular orbital energy level (the same shall apply hereinafter.). In this context, the dihedral angle was calculated using the optimized structure of a trimer (in the formula, n=3 .

A light-emitting device prepared using a composition comprising the polymer (P-1) and a phosphorescence-emitting compound is excellent in luminous efficiency.

<Example 2>

A polymer (P-2) represented by the following formula:

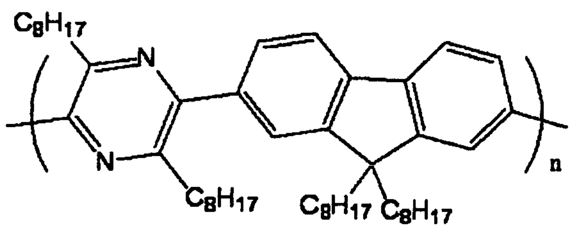

(P-2)

had the lowest triplet excitation energy $T_1$ (1/n=0) of 2.9 eV as an extrapolated value at n=∞ and an absolute value of the lowest unoccupied molecular orbital energy level $E_{LUMO}$ (1/n=0) of 2.1 eV. A simplified repeating unit (M-2a) shown below was used in the calculation. The pyrazine ring and the partial structure (in the present Example, the fluorene skeleton) adjacent to the pyrazine ring formed a dihedral angle of 62° therebetween.

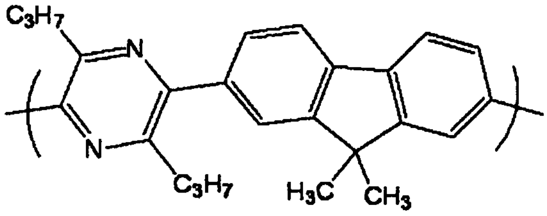

(M-2a)

A light-emitting device prepared using a composition comprising the polymer (P-2) and a phosphorescence-emitting compound is excellent in luminous efficiency.

<Example 3>

A polymer (P-3) substantially represented by the following formula:

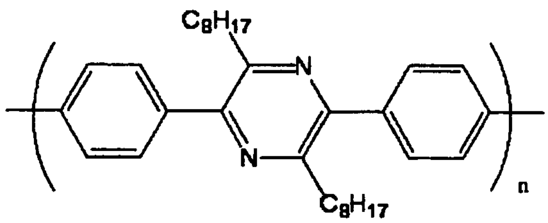

(P-3)

had the lowest triplet excitation energy $T_1$ (1/n=0) of 2.88 eV to 2.9 eV as an extrapolated value at $n=\pi$ and an absolute value of the lowest unoccupied molecular orbital energy level $E_{LUMO}$ (1/n=0) of 2.3 eV. The pyrazine ring and the partial structure (in the present Example, the benzene ring) adjacent to the pyrazine ring formed a dihedral angle of 49° therebetween. Moreover, the benzene ring and the benzene ring adjacent thereto formed a dihedral angle of 44° therebetween. A simplified repeating unit (M-3a) shown below was used in the calculation.

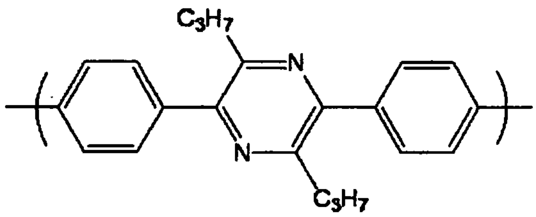

(M-3a)

The polymer substantially represented by the formula (P-3) comprises two types of repeating units. A simplified repeating unit (M-3b) shown below was also possible in addition to the simplified repeating unit (M-3a). As a result of calculation using the repeating unit (M-3b), the polymer had the lowest triplet excitation energy $T_1$ (1/n=0) of 2.89 eV to 2.9 eV as an extrapolated value at $n=\infty$ and an absolute value of the lowest unoccupied molecular orbital energy level $E_{LUMO}$ (1/n=0) of 2.3 eV. For the lowest triplet excitation energy $T_1$ as an extrapolated value at $n=\infty$, the calculated value (2.88 eV) obtained using the repeating unit (M-3a) was smaller than the calculated value (2.89 eV) obtained using the repeating unit (M-3b). Therefore, the lowest triplet excitation energy and the absolute value of the lowest unoccupied molecular orbital energy level $E_{LUMO}$ of the polymer (P-3) were set to the calculated values 2.9 eV and 2.3 eV, respectively, obtained using the repeating unit (M-3a).

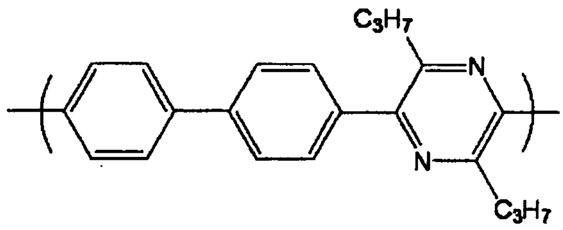

(M－3 b)

<Example 4>

A 0.05 wt% THF solution of a phosphorescence-emitting compound (MC-1) represented by the following formula:

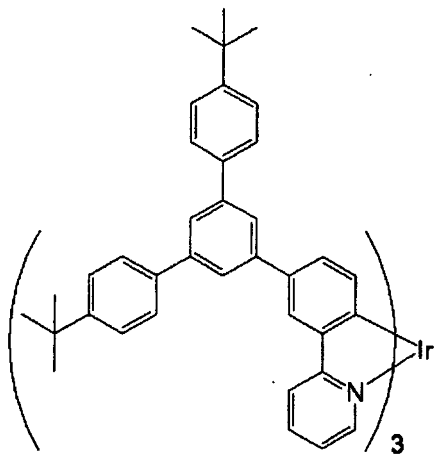

(MC-1)

was mixed with an approximately 5-fold weight of an approximately 1 wt% THF solution of a compound represented by the following formula (6-1):

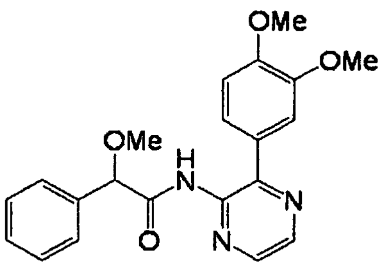

(6-1)

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.
The compound represented by the formula (6-1) had the lowest triplet excitation energy $T_1$ of 3.4 eV and an absolute value of a lowest unoccupied molecular orbital energy level $E_{LUMO}$ of 2.0 eV. Moreover, the pyrazine ring and the partial structure (in the present Example, the benzene ring) adjacent to the pyrazine ring formed a dihedral angle of 59° therebetween.
In this context, the compound represented by the formula (6-1) was synthesized according to a method described in JP-A-2004-292432. Moreover, the compound represented by the formula (MC-1) was synthesized according to a method described in WO02/066552.

<Example 5>

A 0.05 wt% THF solution of the phosphorescence-emitting compound (MC-1) was mixed with an approximately 5-fold weight of an approximately 1 wt% THF solution of a compound represented by the following formula (6-2):

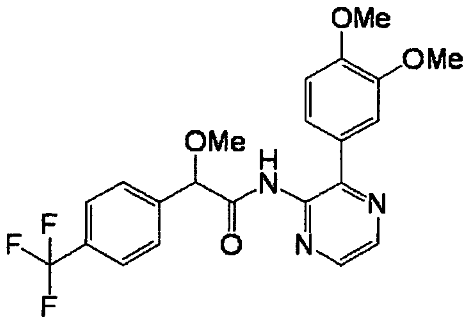

(6-2)

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.

The compound represented by the formula (6-2) had the lowest triplet excitation energy $T_1$ of 3.4 eV and an absolute value of a lowest unoccupied molecular orbital energy level $E_{LUMO}$ of 2.1 eV. Moreover, the pyrazine ring and the partial structure (in the present Example, the benzene ring) adjacent to the pyrazine ring formed a dihedral angle of 60° therebetween.

In this context, the compound represented by the formula (6-2) was synthesized according to a method described in JP-A-2004-292432.

<Example 6>

A 0.05 wt% THF solution of the phosphorescence-emitting compound (MC-1) was mixed with an approximately 5-fold weight of an approximately 1 wt% THF solution of a compound represented by the following formula (6-3):

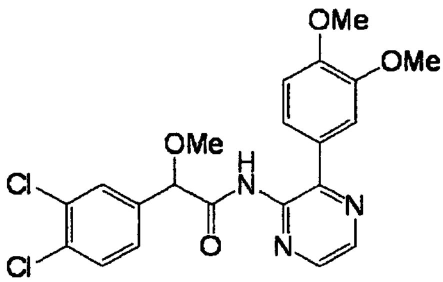

(6-3)

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.

The compound represented by the formula (6-3) had the lowest triplet excitation energy $T_1$ of 3.3 eV and an absolute value of a lowest unoccupied molecular orbital energy level $E_{LUMO}$ of 2.2 eV. Moreover, the pyrazine ring and the partial structure (in the present Example, the benzene ring) adjacent to the pyrazine ring formed a dihedral angle of 59° therebetween.

In this context, the compound represented by the formula (6-3) was synthesized according to a method described in JP-A-2004-292432.

<Example 7>

A 0.05 wt% THF solution of the phosphorescence-emitting compound (MC-1) was mixed with an approximately 5-fold weight of an approximately 1 wt% THF solution of a compound represented by the following formula (6-4):

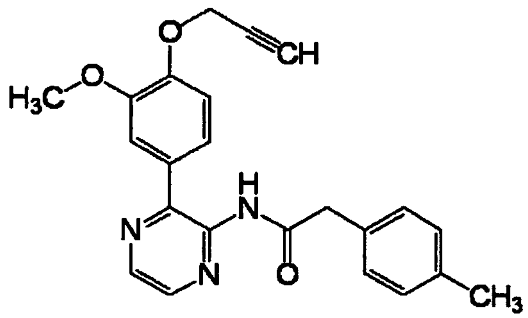

(6-4)

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.
The compound represented by the formula (6-4) had the lowest triplet excitation energy $T_1$ of 3.4 eV and an absolute value of a lowest unoccupied molecular orbital energy level $E_{LUMO}$ of 2.0 eV. Moreover, the pyrazine ring and the partial structure (in the present Example, the benzene ring) adjacent to the pyrazine ring formed a dihedral angle of 62° therebetween.
In this context, the compound represented by the formula (6-4) was synthesized according to a method described in JP-A-2004-292432.

<Example 8>

A 0.05 wt% THF solution of the phosphorescence-emitting compound (MC-1) was mixed with an approximately 5-fold weight of an approximately 1 wt% THF solution of a compound represented by the following formula (6-5):

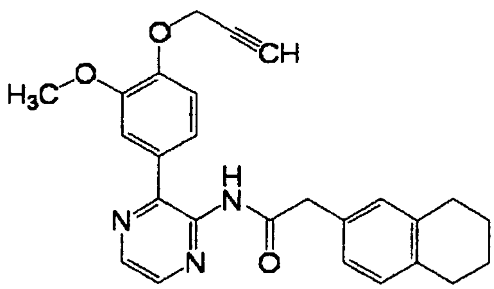

(6-5)

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.
The compound represented by the formula (6-5) had the lowest triplet excitation energy $T_1$ of 3.4 eV and an absolute value of a lowest unoccupied molecular orbital energy level $E_{LUMO}$ of 2.0 eV. Moreover, the pyrazine ring and the partial structure (in the present Example, the benzene ring) adjacent to the pyrazine ring formed a dihedral angle of 62° therebetween.
In this context, the compound represented by the formula (6-5) was synthesized according to a method described in JP-A-2004-292432.

<Example 9>

A 0.05 wt% THF solution of the phosphorescence-emitting compound (MC-1) was mixed with an approximately 5-fold weight of an approximately 1 wt% THF solution of a compound represented by the following formula (6-6):

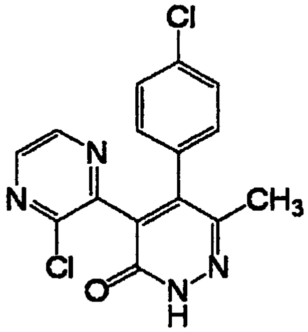

**(6-6)**

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.
The compound represented by the formula (6-6) had the lowest triplet excitation energy $T_1$ of 2.9 eV and an absolute value of a lowest unoccupied molecular orbital energy level $E_{LUMO}$ of 2.4 eV. Moreover, the pyrazine ring and the partial structure (in the present Example, the benzene ring) adjacent to the pyrazine ring formed a dihedral angle of 77° therebetween. Two ring structures other than the pyrazine ring formed a dihedral angle of 89° therebetween.
In this context, the compound represented by the formula (6-6) was synthesized according to a method described in JP-A-2007-254456.

<Example 10>

A 0.05 wt% THF solution of the phosphorescence-emitting compound (MC-1) was mixed with an approximately 5-fold weight of an approximately 1 wt% THF solution of a compound represented by the following formula (6-7):

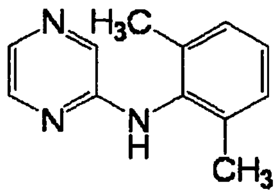

**(6-7)**

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.
The compound represented by the formula (6-7) had the lowest triplet excitation energy $T_1$ of 3.3 eV and an absolute value of a lowest unoccupied molecular orbital energy level $E_{LUMO}$ of 1.5 eV.

<Example 11>

A 0.05 wt% THF solution of the phosphorescence-emitting compound (MC-1) was mixed with an approximately 5-fold weight of an approximately 1 wt% THF solution of a compound represented by the following formula (6-8):

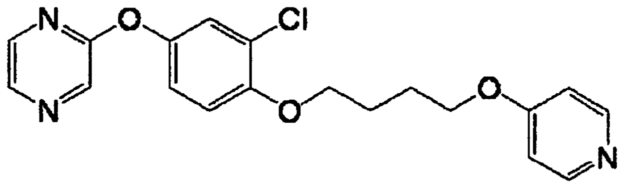

(6-8)

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.
The compound represented by the formula (6-8) had the lowest triplet excitation energy $T_1$ of 3.6 eV and an absolute value of a lowest unoccupied molecular orbital energy level $E_{LUMO}$ of 2.0 eV.

<Example 12>

A 0.05 wt% THF solution of the phosphorescence-emitting compound (MC-1) was mixed with an approximately 5-fold weight of an approximately 1 wt% THF solution of a compound represented by the following formula (6-9):

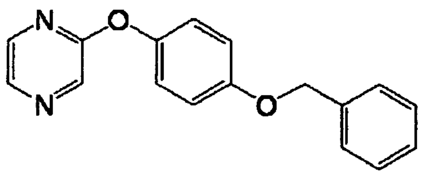

(6-9)

A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.
The compound represented by the formula (6-9) had the lowest triplet excitation energy $T_1$ of 3.6 eV and an absolute value of a lowest unoccupied molecular orbital energy level $E_{LUMO}$ of 1.8 eV.

<Example 13>

A solution was prepared in the same way as in Example 4 except that a phosphorescence-emitting compound (MC-2) shown below was used instead of the phosphorescence-emitting compound (MC-1) in Example 4. The solution was irradiated with UV rays. As a result, blue light emission from the phosphorescence-emitting compound (MC-2, manufactured by American Dye Source, Inc., trade name: ADS065BE) was confirmed.

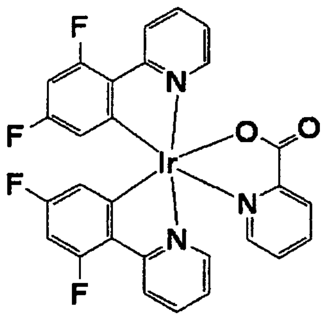

(MC-2)

<Example 14>

A solution was prepared in the same way as in Example 5 except that the phosphorescence-emitting compound (MC-2) shown below was used instead of the phosphorescence-emitting compound (MC-1) in Example 5. The solution was irradiated with UV rays. As a result, blue light emission from the phosphorescence-emitting compound (MC-2) was confirmed.

<Example 15>

A solution was prepared in the same way as in Example 6 except that the phosphorescence-emitting compound (MC-2) shown below was used instead of the phosphorescence-emitting compound (MC-1) in Example 6. The solution was irradiated with UV rays. As a result, blue light emission from the phosphorescence-emitting compound (MC-2) was confirmed.

<Example 16>

A solution was prepared in the same way as in Example 7 except that the phosphorescence-emitting compound (MC-2) shown below was used instead of the phosphorescence-emitting compound (MC-1) in Example 7. The solution was irradiated with UV rays. As a result, blue light emission from the phosphorescence-emitting compound (MC-2) was confirmed.

<Example 17>

A solution was prepared in the same way as in Example 8 except that the phosphorescence-emitting compound (MC-2) shown below was used instead of the phosphorescence-emitting compound (MC-1) in Example 8. The solution was irradiated with UV rays. As a result, blue light emission from the phosphorescence-emitting compound (MC-2) was confirmed.

<Example 18>

A solution was prepared in the same way as in Example 9 except that the phosphorescence-emitting compound (MC-2) shown below was used instead of the phosphorescence-emitting compound (MC-1) in Example 9. The solution was irradiated with UV rays. As a result, blue light emission from the phosphorescence-emitting compound (MC-2) was confirmed.

<Example 19>

A solution was prepared in the same way as in Example 10 except that the phosphorescence-emitting compound (MC-2) shown below was used instead of the phosphorescence-emitting compound (MC-1) in Example 10. The solution was irradiated with UV rays. As a result, blue light emission from the phosphorescence-emitting compound (MC-2) was confirmed.

<Example 20>

A solution was prepared in the same way as in Example 11 except that the phosphorescence-emitting compound (MC-2) shown below was used instead of the phosphorescence-emitting compound (MC-1) in Example 11. The solution was irradiated with UV rays. As a result, blue light emission from the phosphorescence-emitting compound (MC-2) was confirmed.

<Example 21>

A solution was prepared in the same way as in Example 12 except that the phosphorescence-emitting compound (MC-2) shown below was used instead of the phosphorescence-emitting compound (MC-1) in Example 12. The solution was irradiated with UV rays. As a result, blue light emission from the phosphorescence-emitting compound (MC-2) was confirmed.

<Example 24>

2-aminocaprylic acid (10.0 g, 62.8 mmol) and ethylene glycol (100 ml) were placed in atmosphere, heated at 200°C, and stirred for 5 hours. After the completion of reaction, the obtained reaction solution was cooled to room temperature, and 100 ml of water was added thereto. The deposited solid was filtered and washed with diethyl ether to obtain 3,6-dihexyldiketopiperazine represented by the following formula:

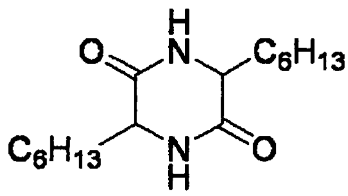

(8.46 g, yield: 44.52%) in a white solid form. LC-MS (APPI)=$[M+H]^+$=283
3,6-dihexyldiketopiperazine (1.00 g, 3.54 mmol) and phosphoryloxy tribromide (3.03 g, 10.62 mmol) were heated to 100°C in an argon atmosphere and stirred for 15 minutes. The obtained mixture was dissolved in 200 mL of chloroform. Then, the organic layer was washed with an aqueous sodium hydroxide solution. After removal of the aqueous phase, the organic phase was dried over anhydrous sodium sulfate. The organic solvent was distilled off under reduced pressure to obtain a crude product. This procedure was performed three times. The obtained crude products were combined and purified by silica gel chromatography (toluene/hexane=2/1) to obtain 2-bromo-3,6-dihexylpyrazine represented by the following formula:

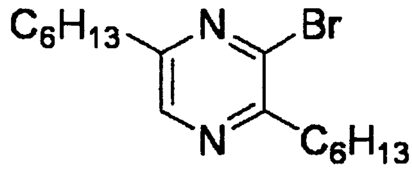

(2.00 g, yield: 57.9%) in an oil form.
NMR $^1H$ ($CDCl_3$) δ: 8.26 (s, 1H), 2.81 (m, 4H), 1.73 (m, 4H), 1.34 (m, 12H), 0.89 (m, 6H).
NMR $^{13}C$ ($CDCl_3$) δ: 156.1, 141.7, 36.4, 34.9, 31.8, 29.7, 29.3, 29.2, 28.3, 22.8, 14.3.
LC-MS (APPI): $[M+H]^+$ = 327
2,7-bis(1,3,2-dioxaborolan-2-yl)-9,9-dioctylfluorene (0.119 g, 0.5 mmol), 2-bromo-3,6-dihexylpyrazine (0.360 g, 1.1 mmol), tetrakis(triphenylphosphine)palladium (12 mg, 0.01 mmol), argon-degassed toluene (2.4 ml) and an argon-degassed 17.5 wt% aqueous solution (1.3 ml) were placed in an argon atmosphere. The mixture was heated to reflux and stirred for 11 hours. To the obtained mixture, 10 mL of toluene was added, and the organic phase was washed with ion-exchanged water. After removal of the aqueous phase, the organic phase was dried over anhydrous sodium sulfate. The organic solvent was distilled off under reduced pressure to obtain a crude product (0.432 g) in a yellow oil form. The obtained crude product was purified by silica gel chromatography (ethyl acetate/hexane=7/93) to obtain a compound represented by the following formula (6-10):

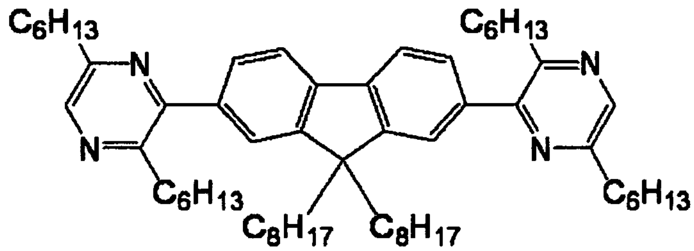

(6-10)

(43.7 mg, yield: 9.9%) in a pale yellow oil form. NMR $^1H$ ($CDCl_3$) δ: 8.38 (s, 2H), 7.86 (d, 2H), 7.55 (d, 2H), 7.48(s, 2H), 2.86 (t, 8H), 2.02 (m, 4H), 1.77 (m, 8H), 1.25 (m, 48H), 0.91 (m, 6H), 0.80 (m, 12H) LC-MS (APCI): $[M+H]^+$ = 883

<Example 25>

A film of a suspension of poly(3,4)ethylenedioxythiophene/polystyrene sulfonic acid (manufactured by Bayer AG, trade name: Baytron P AI4083) was formed as a hole injection layer at a thickness of 65 nm by spin coating on a glass substrate coated with an ITO film at a thickness of 150 nm by a sputtering method. The film was dried at 200°C for 10 minutes on a hot plate. Next, the compound (6-10) thus obtained was dissolved at a concentration of 3.5 wt% in xylene. Moreover, the phosphorescence-emitting compound (MC-1) was also dissolved as a phosphorescence-emitting material at a concentration of 3.5 wt% in xylene. Furthermore, the compound (6-10) thus obtained and the phosphorescence-emitting compound (MC-1) were mixed at a weight ratio of 70/30 to prepare a solution. This mixed solution was applied onto the hole injection layer by spin coating at 1200 rpm. This light-emitting layer had a film thickness of approximately 80 nm. Then, the film was dried at 90°C for 10 minutes in a nitrogen atmosphere containing 10 ppm or lower moisture and oxygen concentrations. Then, barium (approximately 5 nm) and subsequently aluminum (approximately 100 nm) were deposited as a cathode to prepare an EL device. The device comprises ITO/Baytron P (65 nm)/(compound (6-10)/phosphorescence-emitting compound (MC-1)=70/30 (80 nm))/Ba/Al. In this context, the metal deposition was started after the degree of vacuum reached $1\times10^{-4}$ Pa or lower.
A voltage was applied to the obtained device. As a result, the device exhibited green light emission with a peak wavelength of 520 nm derived from the phosphorescence-emitting compound (MC-1). This device had the largest luminous efficiency of 1.80 cd/A.
The compound represented by the formula (6-10) had the lowest triplet excitation energy $T_1$ of 3.0 eV and an absolute value of the lowest unoccupied molecular orbital energy level $E_{LUMO}$ of 1.9 eV. Moreover, the pyrazine ring and the partial structure (in the present Example, the benzene ring) adjacent to the pyrazine ring formed a dihedral angle of 61° therebetween.

<Example 26>

A 10 μl aliquot of the mixed solution comprising the compound (6-10) and the phosphorescence-emitting compound (MC-1) was added dropwise to a slide glass and dried in air to obtain a solid film. This film was irradiated with UV rays at 365 nm. As a result, green light emission from the phosphorescence-emitting compound (MC-1) was confirmed.

<Comparative Example 1>

A polymer (CP-4) represented by the following formula:

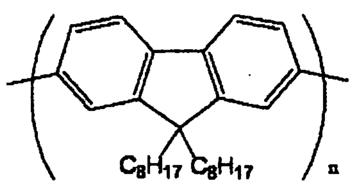

(CP-4)

had the lowest triplet excitation energy $T_1$ (1/n=0) of 2.6 eV as an extrapolated value at n=∞ and an absolute value of the lowest unoccupied molecular orbital energy level $E_{LUMO}$ (1/n=0) of 2.1 eV. A simplified repeating unit (CM-4) shown below was used in the calculation. The adjacent repeating units represented by the formula (CM-4) formed a dihedral angle of 45° therebetween.

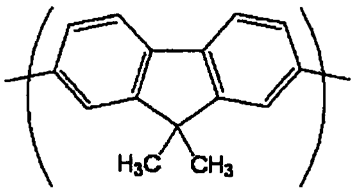

(CM-4).

A light-emitting device prepared using a composition comprising the polymer (CP-4) and a phosphorescence-emitting compound can be confirmed to inferior in luminous efficiency to the light-emitting devices prepared in Examples 1 to 3.

<Comparative Example 2>

A film of a suspension of poly(3,4)ethylenedioxythiophene)/polystyrene sulfonic acid (manufactured by Bayer AG, trade name: Baytron P AI4083) was formed as a hole injection layer at a thickness of 65 nm by spin coating on a glass substrate coated with an ITO film at a thickness of 150 nm by a sputtering method. The film was dried at 200°C for 10 minutes on a hot plate. Next, the compound (CP-4) was dissolved at a concentration of 1.0 wt% in xylene. Moreover, the phosphorescence-emitting compound (MC-1) was also dissolved as a phosphorescence-emitting material at a concentration of 1.0 wt% in xylene. Furthermore, the compound (CP-4) thus obtained and the phosphorescence-emitting compound (MC-1) were mixed at a weight ratio of 70/30 to prepare a solution. This mixed solution was applied onto the hole injection layer by spin coating at 800 rpm. This light-emitting layer had a film thickness of 70 nm. Then, the film was dried at 90°C for 10 minutes in a nitrogen atmosphere containing 10 ppm or lower moisture and oxygen concentrations. Then, barium (approximately 5 nm) and subsequently aluminum (approximately 100 nm) were deposited as a cathode to prepare an organic EL device. The device comprises ITO/Baytron P (65 nm)/(compound (CP-4)/phosphorescence-emitting compound (MC-1)=70/30 (70 nm))/Ba/Al. In this context, the metal deposition was started after the degree of vacuum reached $1\times10^{-4}$ Pa or lower.
A voltage was applied to the obtained device. As a result, peak wavelengths of 440 nm derived from CP-4 and 520 nm derived from MC-1 were observed simultaneously. This device had the largest luminous efficiency of 0.34 cd/A. The compound (CP-4) was synthesized according to a method described in US6512083.

<Comparative Example 3>

A 10 μl aliquot of the mixed solution comprising the compound (CP-4) and the phosphorescence-emitting compound (MC-1) was added dropwise to a slide glass and dried in air to obtain a solid film. This film was irradiated with UV rays at 365 nm. As a result, light emission was darker than that from the compound (CP-4), and difference in color was hardly observed.

<Reference Example>

A 10 μl aliquot of a xylene solution of the compound (CP-4) at a concentration of 1.0 wt% was added dropwise to a slide glass and dried in air to obtain a solid film. This film was irradiated with UV rays at 365 nm. As a result, bright blue light emission from the compound (CP-4) was confirmed.

<Example 27>

1,4-dimethyl-2,5-di(4',4',5',5'-tetramethyl[1',3',2']dioxaborolan-2'-yl)benzene (300 mg), 2,5-dibromopyrazine (452 mg), sodium carbonate (267 mg) and a small amount of bis(triphenylphosphine)palladium(II) dichloride were dissolved in 7 mL of tetrahydrofuran in an argon stream. 4 mL of water was added thereto, and the mixture was stirred at approximately 70°C for approximately 3 hours. After washing with water, chloroform extraction was performed. A polymer was separated from the extract.
A 0.05 wt% THF solution of the phosphorescence-emitting compound (MC-1) was mixed with an approximately

5-fold weight of an approximately 1 wt% THF solution of the polymer. A 10 μl aliquot of the obtained solution was added dropwise to a slide glass and dried in air to obtain a solid film. This film was irradiated with UV rays at 365 nm. As a result, green light emission from the phosphorescence-emitting compound (MC-1) was confirmed.

## Claims

1. A composition comprising a compound having a pyrazine ring structure and a phosphorescence-emitting compound.

2. The composition according to claim 1, wherein the compound having a pyrazine ring structure is a polymer having a pyrazine ring structure.

3. The composition according to claim 1 or 2, wherein the compound having a pyrazine ring structure has a pyrazine ring structure represented by the following general formula (1), (2) or (3):

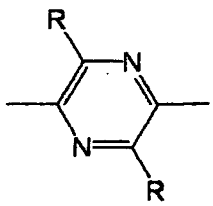

(1)

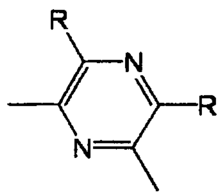

(2)

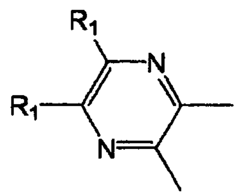

(3)

wherein R and $R_1$ each independently represent a hydrogen atom or a monovalent group, and a plurality of R or $R_1$ may be the same or different.

4. The composition according to claim 1 or 2, wherein the compound having a pyrazine ring structure has a pyrazine ring structure represented by the following general formula (5), (6) or (7):

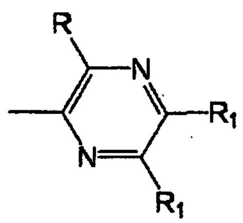

(5)

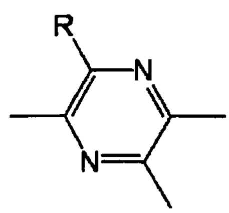

(6)

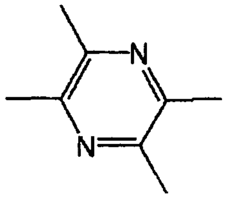

(7)

wherein R and $R_1$ are as defined above, and a plurality of $R_1$ may be the same or different.

**5.** The composition according to claim 3 or 4, wherein at least one of the R is a monovalent group having three or more atoms in total exclusive of hydrogen atoms.

**6.** The composition according to any one of claims 3 to 5, wherein at least one of the R and the $R_1$ is an alkyl group, an alkoxy group, a phenyl group which may have a substituent or a heteroaryl group which may have a substituent.

**7.** The composition according to any one of claims 3 to 6, wherein the compound having a pyrazine ring structure has a pyrazine ring structure represented by the general formula (1), (2), (3), (5), (6) or (7) and a partial structure which is adjacent to the pyrazine ring structure and which has at least two $\pi$-conjugated electrons, wherein the pyrazine ring structure and the partial structure form a dihedral angle of 20° or larger therebetween.

**8.** The composition according to claim 7, wherein the pyrazine ring structure and the partial structure form a dihedral angle of 40° or larger therebetween.

**9.** The composition according to any one of claims 1 to 8, wherein the compound having a pyrazine ring structure has a residue of a compound represented by the following general formula (A-1) or (A-2):

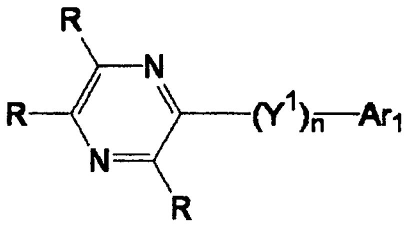

(A-1)

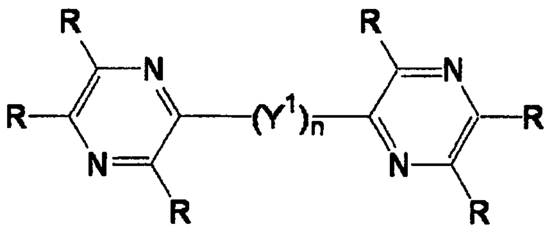

(A-2)

wherein $Y^1$ represents $-C(R^a)(R^b)-$, $-C(=O)-$, $-N(R^c)-$, $-O-$, $-Si(R^d)(R^e)-$, $-P(R^f)-$, $-S-$ or $-S(=O)_2-$; n is an integer of 0 to 5; $Ar_1$ represents a monovalent aryl group which may have a substituent or a monovalent heterocyclic group which may have a substituent; when a plurality of $Y^1$ are present, they may be the same or different; $R^a$ to $R^f$ each independently represent a hydrogen atom or a monovalent group; and R is as defined above, and a plurality of R may be the same or different.

**10.** The composition according to any one of claims 1 to 9, wherein the compound having a pyrazine ring structure has the lowest triplet excitation energy larger than 2.7 eV calculated by a computational chemical approach.

**11.** The composition according to any one of claims 1 to 10, wherein the compound having a pyrazine ring structure has an absolute value of the lowest unoccupied molecular orbital energy level of 2.1 eV or larger calculated by a computational chemical approach.

**12.** The composition according to any one of claims 1 to 11, wherein the lowest triplet excitation energy (ETP) of the compound having a pyrazine ring structure satisfies, with the lowest triplet excitation energy (ETT) of the phosphorescence-emitting compound, the following formula:

$$ETT > ETP-0.20\ (eV).$$

**13.** A polymer having a residue of the phosphorescence-emitting compound and the pyrazine ring structure.

**14.** A light-emitting thin film comprising a composition according to any one of claims 1 to 12 or a polymer according to claim 13.

**15.** An organic semiconductor thin film comprising a composition according to any one of claims 1 to 12 or a polymer according to claim 13.

**16.** A light-emitting device comprising a composition according to any one of claims 1 to 12 or a polymer according to claim 13.

**17.** A planar light source comprising a light-emitting device according to claim 16.

**18.** A segment display device comprising a light-emitting device according to claim 16.

**19.** A dot matrix display device comprising a light-emitting device according to claim 16.

**20.** A liquid-crystal display device comprising a light-emitting device according to claim 16 as a backlight.

**21.** An illumination device comprising a light-emitting device according to claim 16.

**INTERNATIONAL SEARCH REPORT**

International application No.
PCT/JP2007/075068

A. CLASSIFICATION OF SUBJECT MATTER
*C08L65/00*(2006.01)i, *C08G61/12*(2006.01)i, *C08K5/3432*(2006.01)i, *C09K11/06*(2006.01)i, *H01L51/50*(2006.01)i

According to International Patent Classification (IPC) or to both national classification and IPC

B. FIELDS SEARCHED

Minimum documentation searched (classification system followed by classification symbols)
C08L65/00, C08G61/12, C08K5/3432, C09K11/06, H01L51/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| X | JP 2002-317033 A (Fuji Photo Film Co., Ltd.), 31 October, 2002 (31.10.02), Claims; Par. No. [0001], "7" in Par. No. [0031], Par. Nos. [0053] to [0056]; example 6 (Family: none) | 1-8,10-12, 14-21 |
| X<br>A | WO 2006/132354 A1 (Sumitomo Chemical Co., Ltd.), 14 December, 2006 (14.12.06), Claims; "2A-2" in Par. No. [0020], Par. Nos. [0113], [0151] to [0156], [0180] (Family: none) | 1-6,14-21<br>7,8,10-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 March, 2008 (27.03.08) | 08 April, 2008 (08.04.08) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2007/075068

C (Continuation). DOCUMENTS CONSIDERED TO BE RELEVANT

| Category* | Citation of document, with indication, where appropriate, of the relevant passages | Relevant to claim No. |
|---|---|---|
| A | WO 2006/123667 A1 (Mitsubishi Chemical Corp.), 23 November, 2006 (23.11.06), Claims; Par. Nos. [0035] to [0037], [0078] to [0079] & JP 2006-352088 A | 1-8,10-12 14-21 |
| A | JP 2006-344891 A (Fujifilm Holdings Corp.), 21 December, 2006 (21.12.06), Claims; Par. Nos. [0058], [0103] (Family: none) | 1-8,10-12, 14-21 |
| A | JP 2005-225978 A (Toyo Ink Manufacturing Co., Ltd.), 25 August, 2005 (25.08.05), Claims; Par. Nos. [0032], [0045] (Family: none) | 1-8,10-12, 14-21 |

Form PCT/ISA/210 (continuation of second sheet) (April 2007)

**INTERNATIONAL SEARCH REPORT**

International application No.
PCT/JP2007/075068

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: Parts of 7, 8 and 10-12
because they relate to parts of the international application that do not comply with the prescribed requirements to such an extent that no meaningful international search can be carried out, specifically:
See extra sheet.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
2, and parts of 1, 3-8, 10-12 and 14-21

**Remark on Protest** the
- ☐ The additional search fees were accompanied by the applicant's protest and, where applicable, payment of a protest fee.
- ☐ The additional search fees were accompanied by the applicant's protest but the applicable protest fee was not paid within the time limit specified in the invitation.
- ☐ No protest accompanied the payment of additional search fees.

Form PCT/ISA/210 (continuation of first sheet (2)) (April 2007)

INTERNATIONAL SEARCH REPORT

International application No.
PCT/JP2007/075068

Continuation of Box No.II-2 of continuation of first sheet(2)

<Claims 7, 8, 10-12>

In the inventions of these claims, the compound having a pyrazine ring structure and the composition comprising the compound are specified by the dihedral angle between the pyrazine ring structure and a partial structure adjacent to the pyrazine ring structure, the lowest triplet excitation energy of the compound having a pyrazine ring structure, the lowest unoccupied molecular orbital energy level of the compound having a pyrazine ring structure, the lowest triplet excitation energy of the phosphorescent compound or the like.

However, it is unclear as to what compound having what types of structures or what compositions having what kinds of combination of what types of compounds are included within the scope of the compound or the composition having the above-mentioned specified properties, besides those compounds or compositions which are disclosed in the working examples.

Further, since the compounds and compositions having the above-mentioned specified properties excluding those compounds and compositions disclosed in the working examples are unclear as stated above, it requires trial-and-error in an amount beyond that to be expected for a person skilled in the art for obtaining compounds having the above-mentioned specified properties.

Consequently, the above-mentioned claims cannot be considered to be described clearly, and the inventions of the above-mentioned claims cannot be considered to be fully supported or fully disclosed to such an extent that a person skilled in the art can carried out the inventions.

With regard to the inventions of claims which are not clearly described, are not fully supported by the description, or are not clearly or fully described in the description, the search was not made completely.

Form PCT/ISA/210 (extra sheet) (April 2007)

INTERNATIONAL SEARCH REPORT

International application No.
PCT/JP2007/075068

Continuation of Box No.III of continuation of first sheet(2)

The "composition" claimed in claim 1 is "a composition comprising a compound having a pyrazine ring structure and a phosphorescent compound". The "compound having a pyrazine ring structure" may be any compound as long as it has a pyrazine ring structure. Therefore, the invention of claim 1 includes all of the compositions each comprising the compound and a phosphorescent compound as embodiments.

However, these compositions are already known, as disclosed in Document 1 shown below and so on. Therefore, a matter that the composition comprises a compound having a pyrazine ring structure and a phosphorescent compound cannot be regarded as a "special technical feature".

Document 1: WO 2006/123667 A1 (Mitsubishi Chemical Corp.) 23 November, 2006 (23.11.06), claims, paragraph Nos. [0035]-[0037] and [0078]-[0079] & JP 2006-352088 A

Consequently, there is no technical relationship among the embodiments included within the scope of the invention of claim 1 involving one or more of the same or corresponding special technical features, and these embodiments cannot be considered to be so related as to form a single general inventive concept.

The invention of claim 13 relates to "a polymer". Therefore, it cannot be considered that the invention of claim 13 and the invention of claim 1 which relates to "a composition" are so related as to form a single general inventive concept.

As for the "compound having a pyrazine ring structure" recited in claim 1, the polymers (P-1) to (P-3), (6-1) to (6-10) and the polymer (Example 27) are specifically mentioned in the working examples. Therefore, the inventions of claims 1-21 include the following different seven groups of inventions.

(1) The inventions relating to the compositions wherein the "compound having a pyrazine ring structure" is a polymer having a pyrazine structure and selected from the polymers (P-1) to (P-3) and the polymer mentioned in Example 27 (1st group of inventions): claim 2 and parts of claims 1, 3-8, 10-12 and 14-21.

(2) The inventions relating to the compositions wherein the "compound having a pyrazine ring structure" is a low-molecular-weight compound having a pyrazine structure and selected from the compounds (6-1) to (6-10). The inventions are categorized into the following groups depending on their commonalities in chemical structures of the compounds: (6-1) to (6-5); (6-6); (6-7); (6-8) to (6-9); and (6-10) (2nd to 6th groups of inventions): claim 9 and parts of claims 1, 3-8, 10-12 and 14-21.

(3) The inventions relating to the polymer recited in claim 13 (7th group of inventions): claim 13 and parts of claims 14-21.

This international search report was prepared with regard to the 1st group of inventions, namely claim 2 and parts of claims 1, 3-8, 10-12 and 14-21.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002050483 A
- JP 2002241455 A
- WO 2002066552 A
- WO 2004020504 A
- WO 2004020448 A
- JP 63070257 A
- JP 63175860 A
- JP 2135359 A
- JP 2135361 A
- JP 2209988 A
- JP 3037992 A
- JP 3152184 A
- JP 2005126686 A
- JP 2004292432 A
- WO 02066552 A
- JP 2007254456 A
- US 6512083 B


### Non-patent literature cited in the description


- *APPLIED PHYSICS LETTERS,* 2002, vol. 80 (13), 2308
- *Nature,* 1998, vol. 395, 151
- *Appl. Phys, Lett.,* 1999, vol. 75 (1), 4
- Organic Light-Emitting Materials and Devices IV. *Proc. SPIE-Int. Soc. Opt. Eng.,* 2001, vol. 4105, 119
- *J. Am. Chem. Soc.,* 2001, vol. 123, 4304
- *Appl. Phys. Lett.,* 1997, vol. 71 (18), 2596
- *Syn. Met.,* 1998, vol. 94 (1), 103
- *Syn. Met.,* 1999, vol. 99 (2), 1361
- *Adv. Mater.,* 1999, vol. 11 (10), 852
- *Inorg. Chem.,* 2003, vol. 42, 8609
- *Inorg. Chem.,* 2004, vol. 43, 6513
- *Journal of the SID,* 2003, vol. 11/1, 161